# FASCICULE DE BREVET EUROPEEN

(11) **EP 2 387 581 B1**
(45) Date de publication et mention de la délivrance du brevet: **08.10.2014**
(21) Numéro de dépôt: 10703318.5
(22) Date de dépôt: 15.01.2010
(51) Int. Cl.: C07K 14/435, A61K 38/17, C12N 15/63, C12N 15/12, A61P 25/04

(54) **IDENTIFICATION DE NOUVELLES TOXINES ANTAGONISTES DE CANAUX CALCIQUES TYPE-T A VISEE ANALGESIQUE**
IDENTIFIKATION NEUER ANTAGONISTISCHER TOXINE DES KALZIUMKANALS VOM T-TYP FÜR ANALGETISCHE ZWECKE
IDENTIFICATION OF NOVEL ANTAGONIST TOXINS OF T-TYPE CALCIUM CHANNEL FOR ANALGESIC PURPOSES

(30) Priorité: 15.01.2009 FR 0900174
(43) Date de publication de la demande: 23.11.2011
(73) Titulaire: Centre National de la Recherche Scientifique (CNRS), 75794 Paris Cedex 16 (FR); Universite De Nice-Sophia Antipolis, 06108 Nice Cedex 02 (FR)
(72) Inventeur: BOURINET, Emmanuel, F-34270 Saint Mathieu de Tréviers (FR); ESCOUBAS, Pierre, F-06560 Valbonne (FR); MARGER, Fabrice, F-34430 Saint-Jean de Vedas (FR); NARGEOT, Joël, F-34090 Montpellier (FR); LAZDUNSKI, Michel, F-06000 Nice (FR)
(74) Mandataire: Novagraaf Technologies
(86) Numéro de dépôt international: PCT/FR2010/000037
(87) Numéro de publication internationale: WO 2010/081971

(56) Documents cités:
- US-A- 5 756 663
- US-A- 5 776 896
- MIDDLETON RICHARD E ET AL: "Two tarantula peptides inhibit activation of multiple sodium channels." BIOCHEMISTRY, vol. 41, no. 50, 17 décembre 2002 (2002-12-17), pages 14734-14747, XP002537028 ISSN: 0006-2960
- KRAUS R L ET AL: "Modulation of alpha1G and alpha1C Ca channels by the spider toxin ProTx-II" SOCIETY FOR NEUROSCIENCE ABSTRACTS, vol. 26, no. 1-2, 2000, pages Abstract No.-234.14, XP009120110 & 30TH ANNUAL MEETING OF THE SOCIETY OF NEUROSCIENCE; NEW ORLEANS, LA, USA; NOVEMBER 04-09, 2000 ISSN: 0190-5295

### SEQUENCE LISTING

<110> CNRS UniversitÈ de Nice Sophia-Antipolis BOURINET, Emmanuel ESCOUBAS, pierre MARGER, Fabrice NARGEOT, Joîl LAZDUNSKI, Michel
<120> Identification d'une nouvelle toxine antagoniste de canaux calciques de type T a visee analgÈsique
<130> BIP133347PC00
<150> FR09001274
   <151> 2009-01-15
<160> 6
<170> PatentIn version 3.5
<210> 1
   <211> 28
   <212> PRT
   <213> Artificial
<220>
   <223> peptide analgÈsique
<400> 1
   Tyr Cys Gln Lys Phe Leu Trp Thr Cys Asp Ser Glu Arg Pro Cys Cys
   1               5                   10                  15
   Glu Gly Leu Val Cys Arg Leu Trp Cys Lys Ile Asn
               20                  25
<210> 2
   <211> 84
   <212> DNA
   <213> Artificial
<220>
   <223> sequence d'acides nuclÈiques codant pour le peptide analgÈsique
<400> 2
   tattgccaga aatttctgtg gacctgcgat agcgaacgcc cgtgctgcga aggcctggtg      60
   tgccgcctgt ggtgcaaaat taac                                              84
<210> 3
   <211> 84
   <212> DNA
   <213> Artificial
<220>
   <223> sequence codant pour un peptide anagÈsique
<220>
   <221> misc_feature
   <222> (3)..(3)
   <223> Y est C ou T
<220>
   <221> misc_feature
   <222> (6)..(6)
   <223> Y est C ou T
<220>
   <221> misc_feature
   <222> (9)..(9)
   <223> R est A ou G
<220>
   <221> misc_feature
   <222> (12)..(12)
   <223> R est A ou G
<220>
   <221> misc_feature
   <222> (15)..(16)
   <223> Y est C ou T
<220>
   <221> misc_feature
   <222> (18)..(18)
   <223> n is a, c, g, or t
<220>
   <221> misc_feature
   <222> (24)..(24)
   <223> n is a, c, g, or t
<220>
   <221> misc_feature
   <222> (27)..(27)
   <223> Y est C ou T
<220>
   <221> misc_feature
   <222> (30)..(30)
   <223> Y est C ou T
<220>
   <221> misc_feature
   <222> (31)..(31)
   <223> W est A ou T
<220>
   <221> misc_feature
   <222> (32)..(32)
   <223> S est C ou G
<220>
   <221> misc_feature
   <222> (33)..(33)
   <223> n is a, c, g, or t
<220>
   <221> misc_feature
   <222> (36)..(36)
   <223> R est A ou G
<220>
   <221> misc_feature
   <222> (37)..(37)
   <223> M est A ou c
<220>
   <221> misc_feature
   <222> (39)..(39)
   <223> n is a, c, g, or t
<220>
   <221> misc_feature
   <222> (42)..(42)
   <223> n is a, c, g, or t
<220>
   <221> misc_feature
   <222> (45)..(45)
   <223> Y est C ou T
<220>
   <221> misc_feature
   <222> (48)..(48)
   <223> Y est C ou T
<220>
   <221> misc_feature
   <222> (51)..(51)
   <223> R est A ou G
<220>
   <221> misc_feature
   <222> (54)..(54)
   <223> n is a, c, g, or t
<220>
   <221> misc_feature
   <222> (55)..(55)
   <223> Y est C ou T
<220>
   <221> misc_feature
   <222> (57)..(57)
   <223> n is a, c, g, or t
<220>
   <221> misc_feature
   <222> (60)..(60)
   <223> n is a, c, g, or t
<220>
   <221> misc_feature
   <222> (63)..(63)
   <223> Y est C ou T
<220>
   <221> misc_feature
   <222> (64)..(64)
   <223> M est A ou c
<220>
   <221> misc_feature
   <222> (66)..(66)
   <223> n is a, c, g, or t
<220>
   <221> misc_feature
   <222> (67)..(67)
   <223> Y est C ou T
<220>
   <221> misc_feature
   <222> (69)..(69)
   <223> n is a, c, g, or t
<220>
   <221> misc_feature
   <222> (75)..(75)
   <223> Y est C ou T
<220>
   <221> misc_feature
   <222> (78)..(78)
   <223> R est A ou G
<220>
   <221> misc_feature
   <222> (81)..(81)
   <223> H est A ou C
<220>
   <221> misc_feature
   <222> (84)..(84)
   <223> Y est C ou T
<400> 3
   taytgycara arttyytntg gacntgygay wsngarmgnc cntgytgyga rggnytngtn 60
   tgymgnytnt ggtgyaarat haay 84
<210> 4
   <211> 2481
   <212> DNA
   <213> Artificial
<220>
   <223> plasmide pT7-7
<220>
   <221> misc_feature
   <222> (428)..(428)
   <223> n is a, c, g, or t
<220>
   <221> misc_feature
   <222> (435)..(435)
   <223> n is a, c, g, or t
<400> 4
   aattctcatg tttgacagct tatcatcgat aagcttgggc tgcaggtcga ctctagagga 60
   tccccgggcg cgaattctag ccatatgtat atctccttct taaagttaaa caaaattatt 120
   tctagaggga aaccgttgtg gtctccctat agtgagtcgt attaatttcg aagtctatca 180
   gaagttcgaa tcgctgggcc tcgcgcgttt cggtgatgac ggtgaaaacc tctgacacat 240
   gcagctcccg gagacggtca cagcttgtct gtaagcggat gccgggagca gacaagcccg 300
   tcagggcgcg tcagcgggtg ttggcgggtg tcggggcgca gccatgaccc agtcacgtag 360
   cgatagcgga gtgtatatac tggcttaact atgcggcatc agagcagatt gtactgagag 420
   tgcaccanag atctntgcgg tgtgaaatac cgcacagatg cgtaaggaga aaataccgca 480
   tcaggcgctc ttccgcttcc tcgctcactg actcgctgcg ctcggtcgtt cggctgcggc 540
   gagcggtatc agctcactca aaggcggtaa tacggttatc cacagaatca ggggataacg 600
   caggaaagaa catgtgagca aaaggccagc aaaaggccag gaaccgtaaa aaggccgcgt 660
   tgctggcgtt tttccatagg ctccgccccc ctgacgagca tcacaaaaat cgacgctcaa 720
   gtcagaggtg gcgaaacccg acaggactat aaagatacca ggcgtttccc cctggaagct 780
   ccctcgtgcg ctctcctgtt ccgaccctgc cgcttaccgg atacctgtcc gcctttctcc 840
   cttcgggaag cgtggcgctt tctcaatgct cacgctgtag gtatctcagt tcggtgtagg 900
   tcgttcgctc caagctgggc tgtgtgcacg aaccccccgt tcagcccgac cgctgcgcct 960
   tatccggtaa ctatcgtctt gagtccaacc cggtaagaca cgacttatcg ccactggcag 1020
   cagccactgg taacaggatt agcagagcga ggtatgtagg cggtgctaca gagttcttga 1080
   agtggtggcc taactacggc tacactagaa ggacagtatt tggtatctgc gctctgctga 1140
   agccagttac cttcggaaaa agagttggta gctcttgatc cggcaaacaa accaccgctg 1200
   gtagcggtgg tttttttgtt tgcaagcagc agattacgcg cagaaaaaaa ggatctcaag 1260
   aagatccttt gatcttttct acggggtctg acgctcagtg gaacgaaaac tcacgttaag 1320
   ggattttggt catgagatta tcaaaaagga tcttcaccta gatcctttta aattaaaaat 1380
   acgaaagggc ctcgtgatac gcctattttt ataggttaat gtcatgataa taatggtttc 1440
   ttagacgtca ggtggcactt ttcggggaaa tgtgcgcgga acccctattt gtttattttt 1500
   ctaaatacat tcaaatatgt atccgctcat gagacaataa ccctgataaa tgcttcaata 1560
   atattgaaaa aggaagagta tgagtattca acatttccgt gtcgccctta ttcccttttt 1620
   tgcggcattt tgccttcctg tttttgctca cccagaaacg ctggtgaaag taaaagatgc 1680
   tgaagatcag ttgggtgcac gagtgggtta catcgaactg gatctcaaca gcggtaagat 1740
   ccttgagagt tttcgccccg aagaacgttt tccaatgatg agcactttta aagttctgct 1800
   atgtggcgcg gtattatccc gtgttgacgc cgggcaagag caactcggtc gccgcataca 1860
   ctattctcag aatgacttgg ttgagtactc accagtcaca gaaaagcatc ttacggatgg 1920
   catgacagta agagaattat gcagtgctgc cataaccatg agtgataaca ctgcggccaa 1980
   cttacttctg acaacgatcg gaggaccgaa ggagctaacc gcttttttgc acaacatggg 2040
   ggatcatgta actcgccttg atcgttggga accggagctg aatgaagcca taccaaacga 2100
   cgagcgtgac accacgatgc ctgtagcaat ggcaacaacg ttgcgcaaac tattaactgg 2160
   cgaactactt actctagctt cccggcaaca attaatagac tggatggagg cggataaagt 2220
   tgcaggacca cttctgcgct cggcccttcc ggctggctgg tttattgctg ataaatctgg 2280
   agccggtgag cgtgggtctc gcggtatcat tgcagcactg gggccagatg gtaagccctc 2340
   ccgtatcgta gttatctaca cgacggggag tcaggcaact atggatgaac gaaatagaca 2400
   gatcgctgag ataggtgcct cactgattaa gcattggtaa ctgtcagacc aagtttactc 2460
   atatatactt tagattgatt t 2481
<210> 5
   <211> 4969
   <212> DNA
   <213> Artificial
<220>
   <223> plasmide pGex-4-T1
<400> 5
   acgttatcga ctgcacggtg caccaatgct tctggcgtca ggcagccatc ggaagctgtg 60
   gtatggctgt gcaggtcgta aatcactgca taattcgtgt cgctcaaggc gcactcccgt 120
   tctggataat gttttttgcg ccgacatcat aacggttctg gcaaatattc tgaaatgagc 180
   tgttgacaat taatcatcgg ctcgtataat gtgtggaatt gtgagcggat aacaatttca 240
   cacaggaaac agtattcatg tcccctatac taggttattg gaaaattaag ggccttgtgc 300
   aacccactcg acttcttttg gaatatcttg aagaaaaata tgaagagcat ttgtatgagc 360
   gcgatgaagg tgataaatgg cgaaacaaaa agtttgaatt gggtttggag tttcccaatc 420
   ttccttatta tattgatggt gatgttaaat taacacagtc tatggccatc atacgttata 480
   tagctgacaa gcacaacatg ttgggtggtt gtccaaaaga gcgtgcagag atttcaatgc 540
   ttgaaggagc ggttttggat attagatacg gtgtttcgag aattgcatat agtaaagact 600
   ttgaaactct caaagttgat tttcttagca agctacctga aatgctgaaa atgttcgaag 660
   atcgtttatg tcataaaaca tatttaaatg gtgatcatgt aacccatcct gacttcatgt 720
   tgtatgacgc tcttgatgtt gttttataca tggacccaat gtgcctggat gcgttcccaa 780
   aattagtttg ttttaaaaaa cgtattgaag ctatcccaca aattgataag tacttgaaat 840
   ccagcaagta tatagcatgg cctttgcagg gctggcaagc cacgtttggt ggtggcgacc 900
   atcctccaaa atcggatctg gttccgcgtg gatccccgga attcccgggt cgactcgagc 960
   ggccgcatcg tgactgactg acgatctgcc tcgcgcgttt cggtgatgac ggtgaaaacc 1020
   tctgacacat gcagctcccg gagacggtca cagcttgtct gtaagcggat gccgggagca 1080
   gacaagcccg tcagggcgcg tcagcgggtg ttggcgggtg tcggggcgca gccatgaccc 1140
   agtcacgtag cgatagcgga gtgtataatt cttgaagacg aaagggcctc gtgatacgcc 1200
   tatttttata ggttaatgtc atgataataa tggtttctta gacgtcaggt ggcacttttc 1260
   ggggaaatgt gcgcggaacc cctatttgtt tatttttcta aatacattca aatatgtatc 1320
   cgctcatgag acaataaccc tgataaatgc ttcaataata ttgaaaaagg aagagtatga 1380
   gtattcaaca tttccgtgtc gcccttattc ccttttttgc ggcattttgc cttcctgttt 1440
   ttgctcaccc agaaacgctg gtgaaagtaa aagatgctga agatcagttg ggtgcacgag 1500
   tgggttacat cgaactggat ctcaacagcg gtaagatcct tgagagtttt cgccccgaag 1560
   aacgttttcc aatgatgagc acttttaaag ttctgctatg tggcgcggta ttatcccgtg 1620
   ttgacgccgg gcaagagcaa ctcggtcgcc gcatacacta ttctcagaat gacttggttg 1680
   agtactcacc agtcacagaa aagcatctta cggatggcat gacagtaaga gaattatgca 1740
   gtgctgccat aaccatgagt gataacactg cggccaactt acttctgaca acgatcggag 1800
   gaccgaagga gctaaccgct tttttgcaca acatggggga tcatgtaact cgccttgatc 1860
   gttgggaacc ggagctgaat gaagccatac caaacgacga gcgtgacacc acgatgcctg 1920
   cagcaatggc aacaacgttg cgcaaactat taactggcga actacttact ctagcttccc 1980
   ggcaacaatt aatagactgg atggaggcgg ataaagttgc aggaccactt ctgcgctcgg 2040
   cccttccggc tggctggttt attgctgata aatctggagc cggtgagcgt gggtctcgcg 2100
   gtatcattgc agcactgggg ccagatggta agccctcccg tatcgtagtt atctacacga 2160
   cggggagtca ggcaactatg gatgaacgaa atagacagat cgctgagata ggtgcctcac 2220
   tgattaagca ttggtaactg tcagaccaag tttactcata tatactttag attgatttaa 2280
   aacttcattt ttaatttaaa aggatctagg tgaagatcct ttttgataat ctcatgacca 2340
   aaatccctta acgtgagttt tcgttccact gagcgtcaga ccccgtagaa aagatcaaag 2400
   gatcttcttg agatcctttt tttctgcgcg taatctgctg cttgcaaaca aaaaaaccac 2460
   cgctaccagc ggtggtttgt ttgccggatc aagagctacc aactcttttt ccgaaggtaa 2520
   ctggcttcag cagagcgcag ataccaaata ctgtccttct agtgtagccg tagttaggcc 2580
   accacttcaa gaactctgta gcaccgccta catacctcgc tctgctaatc ctgttaccag 2640
   tggctgctgc cagtggcgat aagtcgtgtc ttaccgggtt ggactcaaga cgatagttac 2700
   cggataaggc gcagcggtcg ggctgaacgg ggggttcgtg cacacagccc agcttggagc 2760
   gaacgaccta caccgaactg agatacctac agcgtgagct atgagaaagc gccacgcttc 2820
   ccgaagggag aaaggcggac aggtatccgg taagcggcag ggtcggaaca ggagagcgca 2880
   cgagggagct tccaggggga aacgcctggt atctttatag tcctgtcggg tttcgccacc 2940
   tctgacttga gcgtcgattt ttgtgatgct cgtcaggggg gcggagccta tggaaaaacg 3000
   ccagcaacgc ggccttttta cggttcctgg ccttttgctg gccttttgct cacatgttct 3060
   ttcctgcgtt atcccctgat tctgtggata accgtattac cgcctttgag tgagctgata 3120
   ccgctcgccg cagccgaacg accgagcgca gcgagtcagt gagcgaggaa gcggaagagc 3180
   gcctgatgcg gtattttctc cttacgcatc tgtgcggtat ttcacaccgc ataaattccg 3240
   acaccatcga atggtgcaaa acctttcgcg gtatggcatg atagcgcccg gaagagagtc 3300
   aattcagggt ggtgaatgtg aaaccagtaa cgttatacga tgtcgcagag tatgccggtg 3360
   tctcttatca gaccgtttcc cgcgtggtga accaggccag ccacgtttct gcgaaaacgc 3420
   gggaaaaagt ggaagcggcg atggcggagc tgaattacat tcccaaccgc gtggcacaac 3480
   aactggcggg caaacagtcg ttgctgattg gcgttgccac ctccagtctg gccctgcacg 3540
   cgccgtcgca aattgtcgcg gcgattaaat ctcgcgccga tcaactgggt gccagcgtgg 3600
   tggtgtcgat ggtagaacga agcggcgtcg aagcctgtaa agcggcggtg cacaatcttc 3660
   tcgcgcaacg cgtcagtggg ctgatcatta actatccgct ggatgaccag gatgccattg 3720
   ctgtggaagc tgcctgcact aatgttccgg cgttatttct tgatgtctct gaccagacac 3780
   ccatcaacag tattattttc tcccatgaag acggtacgcg actgggcgtg gagcatctgg 3840
   tcgcattggg tcaccagcaa atcgcgctgt tagcgggccc attaagttct gtctcggcgc 3900
   gtctgcgtct ggctggctgg cataaatatc tcactcgcaa tcaaattcag ccgatagcgg 3960
   aacgggaagg cgactggagt gccatgtccg gttttcaaca aaccatgcaa atgctgaatg 4020
   agggcatcgt tcccactgcg atgctggttg ccaacgatca gatggcgctg ggcgcaatgc 4080
   gcgccattac cgagtccggg ctgcgcgttg gtgcggatat ctcggtagtg ggatacgacg 4140
   ataccgaaga cagctcatgt tatatcccgc cgttaaccac catcaaacag gattttcgcc 4200
   tgctggggca aaccagcgtg gaccgcttgc tgcaactctc tcagggccag gcggtgaagg 4260
   gcaatcagct gttgcccgtc tcactggtga aaagaaaaac caccctggcg cccaatacgc 4320
   aaaccgcctc tccccgcgcg ttggccgatt cattaatgca gctggcacga caggtttccc 4380
   gactggaaag cgggcagtga gcgcaacgca attaatgtga gttagctcac tcattaggca 4440
   ccccaggctt tacactttat gcttccggct cgtatgttgt gtggaattgt gagcggataa 4500
   caatttcaca caggaaacag ctatgaccat gattacggat tcactggccg tcgttttaca 4560
   acgtcgtgac tgggaaaacc ctggcgttac ccaacttaat cgccttgcag cacatccccc 4620
   tttcgccagc tggcgtaata gcgaagaggc ccgcaccgat cgcccttccc aacagttgcg 4680
   cagcctgaat ggcgaatggc gctttgcctg gtttccggca ccagaagcgg tgccggaaag 4740
   ctggctggag tgcgatcttc ctgaggccga tactgtcgtc gtcccctcaa actggcagat 4800
   gcacggttac gatgcgccca tctacaccaa cgtaacctat cccattacgg tcaatccgcc 4860
   gtttgttccc acggagaatc cgacgggttg ttactcgctc acatttaatg ttgatgaaag 4920
   ctggctacag gaaggccaga cgcgaattat ttttgatggc gttggaatt 4969
<210> 6
   <211> 5900
   <212> DNA
   <213> Artificial
<220>
   <223> plasmide pET32
<400> 6
   atccggatat agttcctcct ttcagcaaaa aacccctcaa gacccgttta gaggccccaa 60
   ggggttatgc tagttattgc tcagcggtgg cagcagccaa ctcagcttcc tttcgggctt 120
   tgttagcagc cggatctcag tggtggtggt ggtggtgctc gagtgcggcc gcaagcttgt 180
   cgacggagct cgaattcgga tccgatatca gccatggcct tgtcgtcgtc gtcggtaccc 240
   agatctgggc tgtccatgtg ctggcgttcg aatttagcag cagcggtttc tttcatacca 300
   gaaccgcgtg gcaccagacc agaagaatga tgatgatgat ggtgcatatg gccagaacca 360
   gaaccggcca ggttagcgtc gaggaactct ttcaactgac ctttagacag tgcacccact 420
   ttggttgccg ccacttcacc gtttttgaac agcagcagag tcgggatacc acggatgcca 480
   tatttcggcg cagtgccagg gttttgatcg atgttcagtt ttgcaacggt cagtttgccc 540
   tgatattcgt cagcgatttc atccagaatc ggggcgatca ttttgcacgg accgcaccac 600
   tctgcccaga aatcgacgag gatcgccccg tccgctttga gtacatccgt gtcaaaactg 660
   tcgtcagtca ggtgaataat tttatcgctc atatgtatat ctccttctta aagttaaaca 720
   aaattatttc tagaggggaa ttgttatccg ctcacaattc ccctatagtg agtcgtatta 780
   atttcgcggg atcgagatcg atctcgatcc tctacgccgg acgcatcgtg gccggcatca 840
   ccggcgccac aggtgcggtt gctggcgcct atatcgccga catcaccgat ggggaagatc 900
   gggctcgcca cttcgggctc atgagcgctt gtttcggcgt gggtatggtg gcaggccccg 960
   tggccggggg actgttgggc gccatctcct tgcatgcacc attccttgcg gcggcggtgc 1020
   tcaacggcct caacctacta ctgggctgct tcctaatgca ggagtcgcat aagggagagc 1080
   gtcgagatcc cggacaccat cgaatggcgc aaaacctttc gcggtatggc atgatagcgc 1140
   ccggaagaga gtcaattcag ggtggtgaat gtgaaaccag taacgttata cgatgtcgca 1200
   gagtatgccg gtgtctctta tcagaccgtt tcccgcgtgg tgaaccaggc cagccacgtt 1260
   tctgcgaaaa cgcgggaaaa agtggaagcg gcgatggcgg agctgaatta cattcccaac 1320
   cgcgtggcac aacaactggc gggcaaacag tcgttgctga ttggcgttgc cacctccagt 1380
   ctggccctgc acgcgccgtc gcaaattgtc gcggcgatta aatctcgcgc cgatcaactg 1440
   ggtgccagcg tggtggtgtc gatggtagaa cgaagcggcg tcgaagcctg taaagcggcg 1500
   gtgcacaatc ttctcgcgca acgcgtcagt gggctgatca ttaactatcc gctggatgac 1560
   caggatgcca ttgctgtgga agctgcctgc actaatgttc cggcgttatt tcttgatgtc 1620
   tctgaccaga cacccatcaa cagtattatt ttctcccatg aagacggtac gcgactgggc 1680
   gtggagcatc tggtcgcatt gggtcaccag caaatcgcgc tgttagcggg cccattaagt 1740
   tctgtctcgg cgcgtctgcg tctggctggc tggcataaat atctcactcg caatcaaatt 1800
   cagccgatag cggaacggga aggcgactgg agtgccatgt ccggttttca acaaaccatg 1860
   caaatgctga atgagggcat cgttcccact gcgatgctgg ttgccaacga tcagatggcg 1920
   ctgggcgcaa tgcgcgccat taccgagtcc gggctgcgcg ttggtgcgga catctcggta 1980
   gtgggatacg acgataccga agacagctca tgttatatcc cgccgttaac caccatcaaa 2040
   caggattttc gcctgctggg gcaaaccagc gtggaccgct tgctgcaact ctctcagggc 2100
   caggcggtga agggcaatca gctgttgccc gtctcactgg tgaaaagaaa aaccaccctg 2160
   gcgcccaata cgcaaaccgc ctctccccgc gcgttggccg attcattaat gcagctggca 2220
   cgacaggttt cccgactgga aagcgggcag tgagcgcaac gcaattaatg taagttagct 2280
   cactcattag gcaccgggat ctcgaccgat gcccttgaga gccttcaacc cagtcagctc 2340
   cttccggtgg gcgcggggca tgactatcgt cgccgcactt atgactgtct tctttatcat 2400
   gcaactcgta ggacaggtgc cggcagcgct ctgggtcatt ttcggcgagg accgctttcg 2460
   ctggagcgcg acgatgatcg gcctgtcgct tgcggtattc ggaatcttgc acgccctcgc 2520
   tcaagccttc gtcactggtc ccgccaccaa acgtttcggc gagaagcagg ccattatcgc 2580
   cggcatggcg gccccacggg tgcgcatgat cgtgctcctg tcgttgagga cccggctagg 2640
   ctggcggggt tgccttactg gttagcagaa tgaatcaccg atacgcgagc gaacgtgaag 2700
   cgactgctgc tgcaaaacgt ctgcgacctg agcaacaaca tgaatggtct tcggtttccg 2760
   tgtttcgtaa agtctggaaa cgcggaagtc agcgccctgc accattatgt tccggatctg 2820
   catcgcagga tgctgctggc taccctgtgg aacacctaca tctgtattaa cgaagcgctg 2880
   gcattgaccc tgagtgattt ttctctggtc ccgccgcatc cataccgcca gttgtttacc 2940
   ctcacaacgt tccagtaacc gggcatgttc atcatcagta acccgtatcg tgagcatcct 3000
   ctctcgtttc atcggtatca ttacccccat gaacagaaat cccccttaca cggaggcatc 3060
   agtgaccaaa caggaaaaaa ccgcccttaa catggcccgc tttatcagaa gccagacatt 3120
   aacgcttctg gagaaactca acgagctgga cgcggatgaa caggcagaca tctgtgaatc 3180
   gcttcacgac cacgctgatg agctttaccg cagctgcctc gcgcgtttcg gtgatgacgg 3240
   tgaaaacctc tgacacatgc agctcccgga gacggtcaca gcttgtctgt aagcggatgc 3300
   cgggagcaga caagcccgtc agggcgcgtc agcgggtgtt ggcgggtgtc ggggcgcagc 3360
   catgacccag tcacgtagcg atagcggagt gtatactggc ttaactatgc ggcatcagag 3420
   cagattgtac tgagagtgca ccatatatgc ggtgtgaaat accgcacaga tgcgtaagga 3480
   gaaaataccg catcaggcgc tcttccgctt cctcgctcac tgactcgctg cgctcggtcg 3540
   ttcggctgcg gcgagcggta tcagctcact caaaggcggt aatacggtta tccacagaat 3600
   caggggataa cgcaggaaag aacatgtgag caaaaggcca gcaaaaggcc aggaaccgta 3660
   aaaaggccgc gttgctggcg tttttccata ggctccgccc ccctgacgag catcacaaaa 3720
   atcgacgctc aagtcagagg tggcgaaacc cgacaggact ataaagatac caggcgtttc 3780
   cccctggaag ctccctcgtg cgctctcctg ttccgaccct gccgcttacc ggatacctgt 3840
   ccgcctttct cccttcggga agcgtggcgc tttctcatag ctcacgctgt aggtatctca 3900
   gttcggtgta ggtcgttcgc tccaagctgg gctgtgtgca cgaacccccc gttcagcccg 3960
   accgctgcgc cttatccggt aactatcgtc ttgagtccaa cccggtaaga cacgacttat 4020
   cgccactggc agcagccact ggtaacagga ttagcagagc gaggtatgta ggcggtgcta 4080
   cagagttctt gaagtggtgg cctaactacg gctacactag aaggacagta tttggtatct 4140
   gcgctctgct gaagccagtt accttcggaa aaagagttgg tagctcttga tccggcaaac 4200
   aaaccaccgc tggtagcggt ggtttttttg tttgcaagca gcagattacg cgcagaaaaa 4260
   aaggatctca agaagatcct ttgatctttt ctacggggtc tgacgctcag tggaacgaaa 4320
   actcacgtta agggattttg gtcatgagat tatcaaaaag gatcttcacc tagatccttt 4380
   taaattaaaa atgaagtttt aaatcaatct aaagtatata tgagtaaact tggtctgaca 4440
   gttaccaatg cttaatcagt gaggcaccta tctcagcgat ctgtctattt cgttcatcca 4500
   tagttgcctg actccccgtc gtgtagataa ctacgatacg ggagggctta ccatctggcc 4560
   ccagtgctgc aatgataccg cgagacccac gctcaccggc tccagattta tcagcaataa 4620
   accagccagc cggaagggcc gagcgcagaa gtggtcctgc aactttatcc gcctccatcc 4680
   agtctattaa ttgttgccgg gaagctagag taagtagttc gccagttaat agtttgcgca 4740
   acgttgttgc cattgctgca ggcatcgtgg tgtcacgctc gtcgtttggt atggcttcat 4800
   tcagctccgg ttcccaacga tcaaggcgag ttacatgatc ccccatgttg tgcaaaaaag 4860
   cggttagctc cttcggtcct ccgatcgttg tcagaagtaa gttggccgca gtgttatcac 4920
   tcatggttat ggcagcactg cataattctc ttactgtcat gccatccgta agatgctttt 4980
   ctgtgactgg tgagtactca accaagtcat tctgagaata gtgtatgcgg cgaccgagtt 5040
   gctcttgccc ggcgtcaata cgggataata ccgcgccaca tagcagaact ttaaaagtgc 5100
   tcatcattgg aaaacgttct tcggggcgaa aactctcaag gatcttaccg ctgttgagat 5160
   ccagttcgat gtaacccact cgtgcaccca actgatcttc agcatctttt actttcacca 5220
   gcgtttctgg gtgagcaaaa acaggaaggc aaaatgccgc aaaaaaggga ataagggcga 5280
   cacggaaatg ttgaatactc atactcttcc tttttcaata ttattgaagc atttatcagg 5340
   gttattgtct catgagcgga tacatatttg aatgtattta gaaaaataaa caaatagggg 5400
   ttccgcgcac atttccccga aaagtgccac ctgaaattgt aaacgttaat attttgttaa 5460
   aattcgcgtt aaatttttgt taaatcagct cattttttaa ccaataggcc gaaatcggca 5520
   aaatccctta taaatcaaaa gaatagaccg agatagggtt gagtgttgtt ccagtttgga 5580
   acaagagtcc actattaaag aacgtggact ccaacgtcaa agggcgaaaa accgtctatc 5640
   agggcgatgg cccactacgt gaaccatcac cctaatcaag ttttttgggg tcgaggtgcc 5700
   gtaaagcact aaatcggaac cctaaaggga gcccccgatt tagagcttga cggggaaagc 5760
   cggcgaacgt ggcgagaaag gaagggaaga aagcgaaagg agcgggcgct agggcgctgg 5820
   caagtgtagc ggtcacgctg cgcgtaacca ccacacccgc cgcgcttaat gcgccgctac 5880
   agggcgcgtc ccattcgcca 5900

## Description

### Domaine technique de l'invention

La présente invention se rapporte à une toxine antagoniste de canaux calcique de type T (« T type channels »). En particulier, elle se rapporte à un peptide composant un peptide, aux acides nucléiques codant pour ce peptide, et à l'utilisation dudit peptide comme antagoniste et/ou agoniste inverse des canaux calciques de type T. La présente invention se rapporte également à l'utilisation de ce peptide pour la fabrication d'un médicament.

Elle trouve notamment une application dans le traitement de la douleur, dans les pathologies cardiovasculaires, dans le traitement de la dysfonction érectile et potentiellement dans le traitement des cancers.

Dans la description ci-dessous, les références indiquées « **Réf.** » renvoient à la liste des références présentées après les exemples.

### Etat de la technique

La prise en compte et le traitement de la douleur sont essentiels pour la qualité de vie. La douleur touche un nombre important de personnes. En Europe, on estime que 60 millions de personnes sont concernées chaque année par la douleur, ce qui représente un coût annuel de 1 milliard de dollars en médicaments pour son traitement. Les dépenses effectuées annuellement en médicaments analgésiques peuvent être évaluées à environ 25 milliards de dollars et devraient atteindre 42 milliards en 2010. La douleur se divise en deux catégories: la douleur aigüe et la douleur chronique. La douleur aiguë correspond à une douleur rapide et brève, limitée dans le temps, à l'opposé de la douleur chronique qui est une douleur persistante et qui peut être liée, par exemple à une hyperalgésie.

De nombreuses études concernant l'identification des mécanismes permettant la transmission de la douleur ont été effectuées. La transmission du signal douleur par le système nerveux fait intervenir des récepteurs neuronaux qui transforment le stimulus douloureux en signal électrique. Celui-ci sera alors transmis au système nerveux central par les nerfs, en faisant intervenir des récepteurs moléculaires spécifiques.

Parmi les divers neurones sensoriels, on peut citer les neurones nocicepteurs qui réagissent spécifiquement aux stimuli douloureux.

En raison de leur spécificité, les neurones nocicepteurs possèdent un répertoire unique de canaux ioniques qui sont des protéines responsables de l'excitabilité cellulaire et qui représentent une classe majeure de cibles de médicaments. Ainsi les canaux ioniques spécifiquement exprimés dans les neurones nocicepteurs représentent autant de cibles pour la découverte de nouveaux analgésiques.

Les molécules inhibant la douleur, ou analgésiques, sont connues depuis très longtemps. On peut citer par exemple la classe des opiacés qui agissent sur la signalisation du signal douloureux et stimulent les voies nerveuses inhibitrices de la douleur. D'autres molécules sont utilisées pour traiter les douleurs chroniques. Ce sont par exemple des molécules agissant sur les récepteurs GABA comme le badofène, le diazepan, le tizanidine et le dartrolène.

Les molécules analgésiques utilisées actuellement présentent plusieurs inconvénients. Il est bien connu que les dérivés des opioïdes peuvent provoquer des phénomènes hallucinatoires et des dépressions cardio-respiratoires. Ils sont également source de dépendances, comme par exemple la dépendance à la morphine, la méthadone etc. Enfin le traitement par les opiacés est associé à des effets indésirables comme une constipation sévère.

Il existe également des cas d'accoutumance à ces médicaments, c'est à dire que la dose nécessaire pour obtenir un effet constant doit être augmentée. Cette accoutumance croît dans le temps et entraîne donc la nécessité d'augmenter les doses et peut entraîner l'inefficacité du médicament. En effet, la quantité nécessaire peut devenir supérieure à la dose toxique dudit médicament.

Par exemple, dans le cas de douleurs neuropathiques qui peuvent intervenir dans des cas de diabète, de cancer, de traitements chimiothérapiques, de douleurs inflammatoires chroniques, de cas de syndrome du colon irritable etc., le traitement de la douleur par des analgésiques et antalgiques connus induit fréquemment un phénomène d'accoutumance et donc des problèmes de traitement de ces douleurs.

Il existe donc un réel besoin de trouver de nouvelles molécules analgésiques ou antalgiques palliant ces défauts, inconvénients et obstacles de l'art antérieur afin de réduire les coûts et d'améliorer le traitement de la douleur et le confort des patients.

### Exposé de l'invention

Les inventeurs ont montré par des expériences d'inhibition de l'expression du gène Cacna1h codant pour le canal calcique de type T Caᵥ3.2 présent dans les nocicepteurs que ledit canal est impliqué dans la perception de la douleur.

Les canaux Cav3.2 font partie de la famille des canaux calciques à « bas seuils » ou « type-T » et sont exprimés dans les neurones nocicepteurs

En utilisant une approche de répression génique par injection intrathécale d'ADN antisens chez le rat comme décrit dans Bourinet, E., et al., "Silencing of the CaV3.2 T-type calcium channel gene in sensory neurons demonstrates its major role in nociception". Embo J, 2005. 24(2): p. 315-24 (**Réf 1**)**,** les inventeurs ont démontré que la déplétion des canaux calciques Caᵥ3.2 dans les neurones nocicepteurs d'animaux sains ou en condition de neuropathie entraîne une analgésie spectaculaire. En conséquence, les canaux calcium de type T représentent une cible potentielle pour le développement de nouvelles approches thérapeutiques du traitement de la douleur.

Par ailleurs, il a été démontré que les canaux calciques Caᵥ3.2 sont impliqués dans la prolifération cellulaire (Taylor, J.T., Zeng, X.B., Pottle, J.E., Lee, K., Wang, A.R., Yi, S.G., Scruggs, J.A., Sikka, S.S. and Li, M. (2008) Calcium signaling and T-type calcium channels in cancer cell cycling. World J Gastroenterol, 14, 4984-4991 (**Ref 8**)) et dans la régulation de la tension sanguine (Chen, C.C., Lamping, K.G., Nuno, D.W., Barresi, R., Prouty, S.J., Lavoie, J.L., Cribbs, L.L., England, S.K., Sigmund, C.D., Weiss, R.M., Williamson, R.A., Hill, J.A. and Campbell, K.P. (2003) Abnormal coronary function in mice deficient in alpha1H T-type Ca2+ channels. Science, 302, 1416-1418. (**Ref 9**)).Les canaux calcium de type T représentent une cible potentielle pour le développement de nouvelles approches thérapeutiques du traitement de l'hypertension et des cancers, par exemple les cancers liés à une prolifération anarchique des cellules cancéreuses.

Il peut s'agir de cancers de divers organes par exemple:
- le cancer de la prostate (voir Gackière F, Bidaux G, Delcourt P, Van Coppenolle F, Katsogiannou M, Dewailly E, Bavencoffe A, Van Chuoï-Mariot MT, Mauroy B, Prevarskaya N, Mariot P.(2008) CaV3.2 T-type calcium channels are involved in calcium-dependent sécrétion of neuroendocrine prostate cancer cells. J Biol Chem, 283(15):10162-73 (**Ref 16**),
- gliome (voir Panner A, Cribbs LL, Zainelli GM, Origitano TC, Singh S, Wurster RD. (2005) Variation of T-type calcium channel protein expression affects cell division of cultured tumor cells. Cell Calcium, 37(2):105-19 (**Ref 11**)),
- le carcinome de l'oesophage (voir Lu F, Chen H, Zhou C, Liu S, Guo M, Chen P, Zhuang H, Xie D, Wu S. (2008) T-type Ca2+ channel expression in human esophageal carcinomas: a functional role in proliferation Cell Calcium. 43(1):49-58 (**Ref 12**))
- le cancer du sein (voir_Taylor JT, Huang L, Pottle JE, Liu K, Yang Y, Zeng X, Keyser BM, Agrawal KC, Hansen JB, Li M (2008) Selective blockade of T-type Ca2+ channels suppresses human breast cancer cell proliferation.. Cancer Lett, 18;267(1):116-24 (**Ref 13**).

En particulier une approche pharmacologique utilisant des antagonistes sélectifs du canal Cav3.2 de type T fait l'objet du présent brevet.

L'invention répond précisément à besoin de trouver de nouvelles molécules analgésiques ou antalgiques en fournissant une nouvelle molécule capable notamment d'inhiber un signal douloureux, plus particulièrement une molécule capable d'inhiber les récepteurs responsables de ce signal. Cette molécule est un peptide de séquence: YCQKFLWTCDSERPCCEGLVCRLWCKIN (SEQ ID NO 1).

Par « dérivé », on entend Ces « fragments » ou « dérivés » sont tels qu'ils conservent les propriétés du peptide de la présente invention. Les dérivés ou fragment peuvent également être considérés comme étant des analogues du peptide de la présente invention.

Par exemple, un dérivé peut être le peptide de séquence ID NO1 avec des modifications sur au moins un des acides aminés. Les modifications sont par exemple les suivantes :
- remplacement d'au moins un acide aminé par un autre de la même famille (aromatique, hydrophobe, basique, etc..) ;
- remplacement d'un acide aminé naturel (amino acide L) par le même acide aminé sous forme D ;
- remplacement d'une liaison peptidique entre deux acides aminés par une liaison pseudo-peptidique.
- modification indépendamment des extrémités N et C terminales du peptide.

Ces dérivés pourront comprendre des acides aminés naturels, des acides aminés modifiés, par exemple des sélénocystéines, des acides aminés non-naturels, et/ou des modifications de structure du peptide comme des cyclisations.

Les dérivés peuvent, par exemple, également comprendre des peptides modifiés en vue d'un marquage par des agents comme la biotine, des groupements fluorophores ou un isotope radioactif.

Les modifications des extrémités N ou C terminales peuvent être par exemple l'isoprénylation, la glypiation, la myristoylation, ou la palmitoylation. L'extrémité C terminale peut être également modifiée par exemple par amidation conduisant à la formation d'une fonction amide à l'extrémité C terminale. Des exemples de modifications post-traductionnelles retrouvées dans des peptides sont présentées dans Han KK, Martinage A (1992) Post-translational chemical modification(s) of proteins. Int J Biochem. 24(1):19-28. ; Walsh G, Jefferis R (2006) Post-translational modifications in the context of therapeutic proteins Nature Biotechnology - 24, 1241 - 1252 (**Ref 17**)). Ces modifications sont des modifications qui peuvent être rencontrées dans les toxines d'insectes (voir par exemple Escoubas P. (2006) Molecular diversification in spider venoms: a web of combinatorial peptide libraries. Mol Divers. 10(4):545-54 (**Ref 18**)).

Dans la description qui suit, par peptide de séquence ID NO 1, on entend la séquence peptidique ID NO 1 ou une séquence dérivée de celle-ci.

L'invention répond également au besoin de trouver de nouvelles molécules antihypertenseurs en fournissant une nouvelle molécule capable notamment de diminuer la pression artérielle.

L'invention répond également au besoin de trouver de nouvelles molécules pour le traitement du cancer en fournissant une nouvelle molécule capable notamment d'inhiber la prolifération cellulaire.

La nouvelle molécule de la présente invention est un peptide. Ce peptide a été isolé pour la première fois par les inventeurs à partir du venin d'une araignée du genre *Paraphysa* (Psp3) (Araneae :Theraphosidae).

Aussi, la présente invention se rapporte également à toute séquence en acides nucléiques codant pour le peptide de séquence YCQKFLWTCDSERPCCEGLVCRLWCKIN (SEQ ID NO 1).

L'acide nucléique de la présente invention peut être toute séquence codant pour le peptide de séquence SEQ ID NO 1 compte tenu de la dégénérescence du code génétique. Par exemple, il peut s'agir des acides nucléiques de séquence suivants : dans lesquelles A est adénosine, C est cytidine, G est guanosine T est thymidine et H est A ou C ou T, M est A ou C, N est A ou C ou G ou T, R est A ou G, S est C ou G, W est A ou T et Y est C ou T:

L'invention se rapporte également à un système d'expression comprenant un plasmide et/ou un vecteur d'expression codant pour le peptide SEQ ID NO 1.

Dans la présente le système d'expression peut comprendre au moins une séquence d'acides nucléiques codant pour le peptide de séquence YCQKFLWTCDSERPCCEGLVCRLWCKIN (SEQ ID NO 1).

Par « vecteur d'expression » dans la présente on entend une séquence d'acide nucléique dans laquelle il est possible d'insérer un ou plusieurs fragments d'acides nucléiques que l'on souhaite exprimer dans un hôte. Le vecteur d'expression peut comporter les éléments permettant l'expression de la séquence d'acide nucléique dans l'hôte. Par exemple, le vecteur peut comprendre une séquence promotrice, une séquence codant pour un peptide signal. Les vecteurs peuvent être tout vecteur connu de l'homme du métier.

Par exemple, les vecteurs d'expression peuvent être choisis dans le groupe comprenant les plasmides, les virus, les vecteurs bactériens, les chromosomes artificiels de levure (YAC) et les chromosomes artificiels de bactéries (BAC). Les vecteurs d'expression peuvent être également des vecteurs de surexpression de protéines recombinantes, avec ou sans fusion à une protéine soluble comme, par exemple la GST, ou la thioredoxine ou toute autre protéine de fusion connue de l'homme du métier.

Par exemple, des vecteurs utilisables dans la présente invention peuvent être choisis notamment dans le groupe comprenant le plasmide pT7-7 (SEQ ID NO 4, Figure 5), un plasmide de la série pGEX (SEQ ID NO 5, Figure 6), commercialisé par exemple par la société Pharmacia, ou un plasmide de la série pET32 (SEQ ID NO 6, Figure 7), commercialisé par exemple par la société Novagen.

Un hôte comprenant un vecteur d'expression du peptide de séquence YCQKFLWTCDSERPCCEGLVCRLWCKIN (SEQ ID NO1) est décrit.

Dans la présente par « hôte », on entend toute cellule pouvant être transformée avec un vecteur d'expression ou modifiée génétiquement afin de produire le peptide de l'invention.

La transformation dudit « hôte » peut être réalisée, par exemple par électroporation ou par toute autre méthode connue de l'homme du métier. La séquence codant pour le peptide de séquence ID NO 1 utilisée lors de la transformation peut être libre ou incorporée dans un des vecteurs précités. Après introduction, ladite séquence en acides nucléiques peut être incorporée dans le génome de l'hôte par recombinaison génétique, rester sous forme libre dans l'hôte et/ou rester dans le vecteur, ladite séquence en acides nucléiques étant ensuite reconnue par les protéines et/ou enzymes de l'hôte et exprimée par celui-ci.

L'hôte peut être choisi dans le groupe comprenant les cellules eucaryotes ou procaryotes, par exemple des bactéries comme Escherichia Coli, des levures comme Pichia pastoris, des cellules d'insectes comme les cellules de Drosophila ou de Spodoptera, des cellules de mammifères et tout autre cellule connue de l'homme du métier qui permet la production de peptides et/ou de protéines à partir de vecteurs d'expression.

Les cellules procaryotes ou eucaryotes peuvent, de préférence, permettre la surexpression du peptide pour lequel code le vecteur. Ainsi, toute cellule hôte capable de d'exprimer un vecteur d'expression pour le peptide de l'invention est utilisable, par exemple Escherichia coli.

De manière préférée, les cellules eucaryotes ou procaryotes choisies sont celles qui permettent la surexpression du peptide de l'invention. Par exemple, il s'agit de cellules Sf9 de *Spodoptera,* de celules S2 de Drosophile, de levures de type Pichia pastoris ou de bactéries *Escherichia coli.*

Les inventeurs montrent dans les exemples ci-dessous que le peptide de séquence SEQ ID NO 1 est un antagoniste et ou un antagoniste partiel des canaux de type T.

Aussi la présente invention se rapporte également au peptide de séquence YCQKFLWTCDSERPCCEGLVCRLWCKIN (SEQ ID NO 1) comme antagoniste d'un canal calcique de type T.

Aussi la présente invention se rapporte également au peptide qu'antagoniste d'un canal calcique de type T de séquence en acide aminés :YCQKFLWTCDSERPCCEGLVCRLWCKIN (SEQ ID NO :1).

Par « antagoniste », on entend un agent, par exemple une molécule chimique, une protéine, un peptide etc. qui interagit avec un site récepteur sur le canal et qui empêche ledit canal de fonctionner.

L'antagoniste peut également diminuer l'effet pharmacologique de l'agoniste, il s'agit alors d'un « antagoniste partiel ».

L'invention a également pour objet l'utilisation du peptide de séquence SEQ ID NO 1 comme agoniste inverse d'un canal de type T. Par « agoniste inverse», on entend un agent, par exemple une molécule chimique, une protéine etc. qui interagit avec le même récepteur qu'un agoniste de ce récepteur mais produit l'effet pharmacologique opposé.

En d'autres termes, l'antagoniste, antagoniste partiel ou l'agoniste inverse peut être une substance se fixant sur les mêmes récepteurs cellulaires au niveau de sites identiques ou différents d'une substance de référence, empêchant celle-ci de produire tout ou partie de ses effets habituels ou produisant l'effet inverse.

Dans la présente, par « canaux calcique de type T » on entend les canaux calciques à faible seuil d'activation, c'est à dire ayant un potentiel d'activation compris entre -80 et -20 mV, de préférence entre -70 et -50 mV

Par exemple, des canaux calciques de type T peuvent être les canaux calciques générés par les sous unités Caᵥ3.1, Caᵥ3.2, Caᵥ3.3 et tous leurs variants d'épissage en association avec leurs sous unités régulatrices ou non.

De manière préférée, le peptide est utilisé comme antagoniste et/ou agoniste inverse des canaux calciques de type T.

De manière encore plus préféré, le peptide est un antagoniste et/ou agoniste inverse des canaux calciques Caᵥ3.2 et/ou des canaux calciques Caᵥ3.1 et/ou Caᵥ3.3.

La présente invention se rapporte également au peptide antagoniste d'un canal calcique de type T de séquence en acide aminés :YCQKFLWTCDSERPCCEGLVCRLWCKIN (SEQ ID NO :1), ledit canal étant un canal choisi dans le groupe comprenant le canal Cav3.2 et le canal Cav3.1.

L'invention a également pour objet l'utilisation du peptide de séquence YCQKFLWTCDSERPCCEGLVCRLWCKIN (SEQ ID NO 1) et/ou d'un acide nucléique codant pour le peptide de séquence YCQKFLWTCDSERPCCEGLVCRLWCKIN (SEQ ID NO 1) pour la fabrication d'un médicament. Ce médicament peut être à usage humain ou vétérinaire.

Dans la présente, le peptide peut être associé à tout support pharmacologiquement acceptable afin par exemple d'améliorer sa biodisponibilité, sa solubilité, sa stabilité en solution et/ou la fabrication du médicament.

La présente invention se rapporte également au peptide de séquence en acide aminés : YCQKFLWTCDSERPCCEGLVCRLWCKIN (SEQ ID NO :1), pour son utilisation comme médicament.

La présente invention se rapporte également au peptide de séquence en acide aminés : YCQKFLWTCDSERPCCEGLVCRLWCKIN (SEQ ID NO :1), pour son utilisation dans le traitement de la douleur.

La présente invention se rapporte également au peptide de séquence en acide aminés: YCQKFLWTCDSERPCCEGLVCRLWCKIN (SEQ ID NO :1), pour son utilisation comme médicament analgésique.

Dans la présente par « support pharmaceutiquement acceptable », on entend tout composé connus par l'homme du métier pour être utilisé dans des médicaments. Il peut d'agir, par exemple des composés cité dans l'ouvrage Pharmacie galénique de A. LEHIR (Ed. Masson, 1992 (6 ème édition)) (**Réf 7**).

Dans la présente par « association », on entend une liaison covalente avec le support, une liaison par interactions ioniques, la présence dans une solution du support et du peptide de l'invention.

Le médicament comprenant le peptide de séquence SEQ ID NO 1 peut être formulé sous toute forme permettant son administration à un patient ou à un animal. Il peut s'agir, par exemple, d'une formulation permettant une administration par voie orale, intraveineuse, péridurale, cutanée, ou transdermique.

Dans la présente par « formulation » on entend une formulation injectable (par exemple comme pour l'octaplex (marque enregistrée) ou le synergon (marque déposée)), une formulation orale par exemple un sirop, une poudre (par exemple comme pour l'octaplex (marque enregistrée)), des granules, un comprimé (comme par exemple pour l'estima (marque enregistrée), un comprimé pelliculé (comme par exemple pour le provames (marque déposée)), une gélule, un spray, une formulation locale comme par exemple une crème (comme par exemple pour la crème trophicrème (marque déposée)), une lotion, un gel, une formulation oculaire par exemple un collyre, une formulation pour injection intraveineuse, une formulation injectable par dispositifs d'auto injection type stylo à insuline, un patch transdermique, comprimés disponibles per os, une formulation pour ingestion sublinguale, ou une formulation pour injection péridurale.

Par exemple, il peut également s'agir des différentes formulations pharmaceutiques décrites dans l'ouvrage Pharmacie galénique de A. LEHIR (Ed. Masson, 1992 (6 ème édition)) (**Réf 7**).

Le médicament peut être, par exemple, administré sous forme de gélules, de comprimés, de patchs, de solutions injectables, de suppositoires et/ou de poudres.

La concentration du peptide de l'invention dans le médicament et/ou le produit vétérinaire peut être toute concentration efficace du point de vue pharmaceutique et acceptable par le patient ou l'animal traité comprise par exemple entre 1 nM et 1000nM.

Le médicament comprenant le peptide de l'invention est de préférence administré afin d'obtenir une concentration intraveineuse permettant un effet biologique. Cette concentration plasmatique efficace peut également être déterminée, par exemple en suivant le temps de demi-vie du peptide de l'invention après son injection par voie intraveineuse à un mammifère et mesure de sa concentration par spectrométrie de masse. Le temps de demi-vie correspond au temps nécessaire pour que la concentration dudit médicament soit égale à la moitié de la concentration initiale.

Le médicament et/ou le produit vétérinaire comprenant le peptide de séquence SEQ ID NO 1 peut être conditionné pour une administration une fois par jour, deux fois par jour, trois fois par jour, tous les deux jours, tous les trois jours, une fois par semaine.

Le mode d'administration et la quantité administrée peuvent être adaptés à chaque individu et peuvent être déterminés par le praticien notamment en fonction des caractéristiques physiologiques de l'individu traité.

Dans la présente le médicament peut-être un produit pour le traitement de la douleur, de la migraine, de l'épilepsie, de la maladie de parkinson, de dysrythmies thalamiques, de troubles du sommeil et de la vigilance, de troubles bipolaires, de pathologies cardiovasculaires (hypertrophie cardiaque, hypertension), de pathologies lié à la libération d'aldostérone, de dysfonctions érectiles, et de pathologies cancéreuses

De préférence, le médicament de la présente invention est un produit pour le traitement de la douleur, par exemple un antalgique, un analgésique, un antiallodynique.

De manière préférée, le médicament et/ou le produit vétérinaire de la présente invention est un analgésique.

La présente invention à également pour objet un procédé de synthèse du peptide de séquence YCQKFLWTCDSERPCCEGLVCRLWCKIN (SEQ ID NO 1) comprenant une étape de synthèse chimique sur support solide ou une synthèse par recombinaison génétique.

Le procédé de synthèse dans la présente invention est un procédé de synthèse chimique sur support solide ou une synthèse par recombinaison génétique.

Le procédé de synthèse par recombinaison génétique peut utiliser un système d'expression ou un vecteur tel que défini ci-dessus.

Le procédé de synthèse du peptide de l'invention par recombinaison génétique peut comprendre notamment les étapes suivantes :
- transformer une cellule hôte avec un vecteur d'expression comprenant une séquence d'acide nucléique codant pour le peptide de l'invention,
- cultiver la cellule hôte transformée dans des conditions de culture telles qu'elle fabrique le peptide de l'invention à partir dudit vecteur d'expression, et
- récupérer ledit peptide fabriqué par la cellule hôte.

Les étapes de transformation, de culture, ainsi que de l'isolement du peptide peuvent être réalisées par les techniques habituelles de recombinaison génétique, par exemple des techniques décrites dans le document Sambrook, Fritsch and Maniatis, « Molecular cloning, A laboratory manual », second edition, Cold spring Harbor Laboratory Press, 1989 (**Réf 3**).

Par exemple, le peptide de séquence de SEQ ID NO 1 peut être préparé par les techniques classiques de synthèse chimique en phase solide, par exemple selon la méthodologie de synthèse peptidique sur support solide Fmoc (« Fmoc solid Phase peptide synthesis, a practical approach », publié par W.C. Chan et P. D.White, Oxford university press, 2000 (**Réf 4**)).

D'autres caractéristiques et avantages de l'invention ressortiront à la lecture de la description qui suit, en référence aux figures annexées.

### Brève description des figures

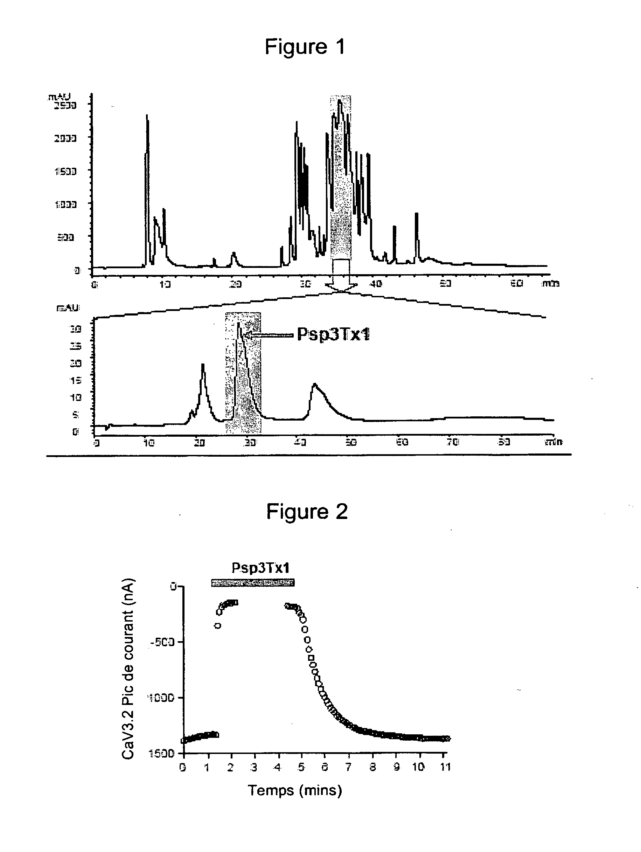

Figure 1 est un diagramme représentant le profil de chromatographie Liquide haute performance (CLHP) du venin de Psp3 (colonne phase inverse) (partie supérieure), exprimé en mAU en fonction du temps. Le diagramme inférieur représente le profil de chromatographie CPLH (colonne échangeuse d'ion) des fractions indiquées par la partie grisée sur le diagramme supérieur. La partie grisée sur le profil CPLH inférieur correspond à un pic pur contenant la toxine Psp3Tx1.
Figure 2 est un diagramme représentant le courant généré par les canaux Caᵥ3.2 en nano ampère (nA) en fonction du temps. La courbe montre l'effet de l'application d'une partie de la Psp3Tx1 purifiée par l'CPLH présenté sur la Figure 1 sur l'activité du canal Caᵥ3.2 exprimé dans des ovocytes de Xénope. Le courant est enregistré dans une solution contenant 5mM Barium comme porteur de charges et il est évoqué par une dépolarisation à -30mV à partir d'un potentiel de repos de -100mV.

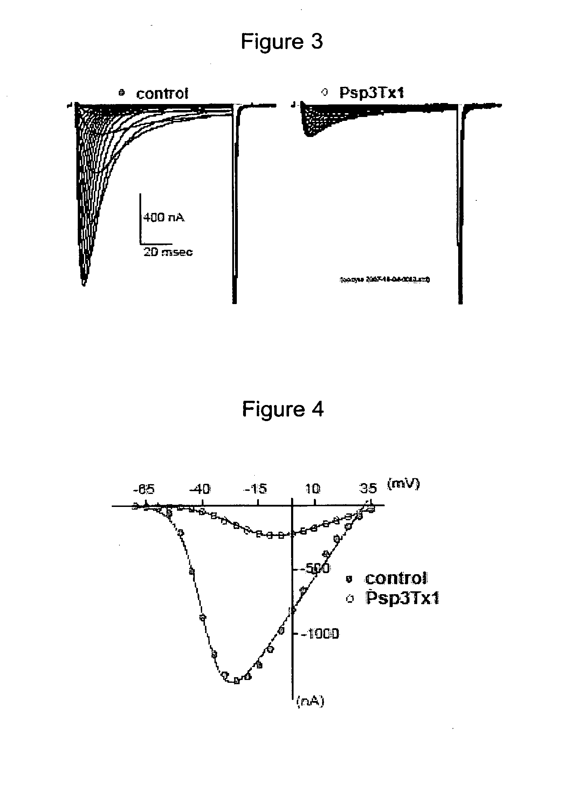

Figure 3 est un diagramme représentant plusieurs enregistrements de courants en nanoAmpère en fonction du temps de type-T générés par la sous-unité CaV3.2 à plusieurs potentiels avant (controle) et pendant l'application de la Psp3Tx1 (Psp3TX1).
Figure 4 est un diagramme représentant des relations courant-potentiel pour les enregistrements présentés dans la figure 3. L'abscisse représentant le courant en nanoAmpère en fonction du potentiel en milliVolte (mV).

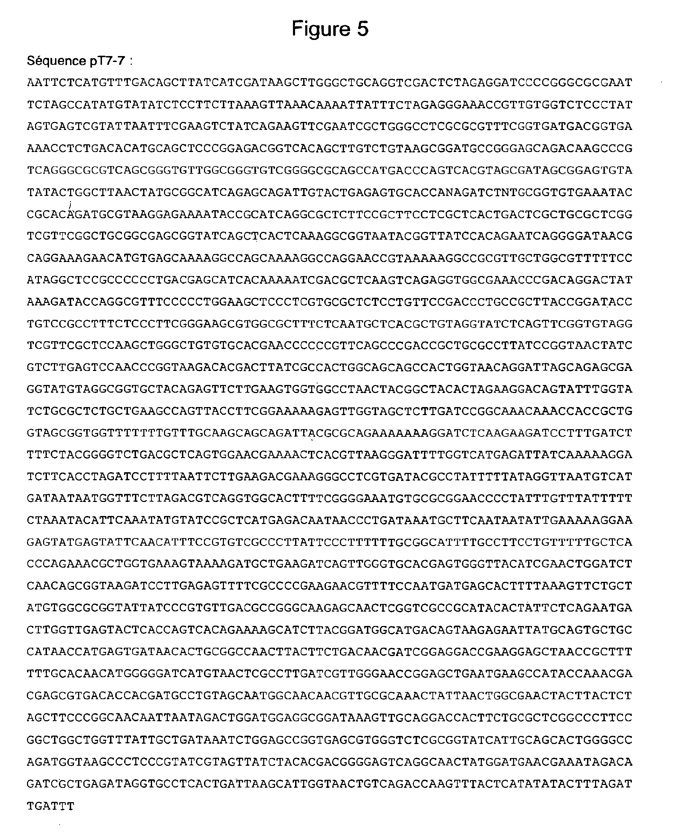

Figure 5 : séquence du plasmide pT7-7 (SEQ ID NO 4).

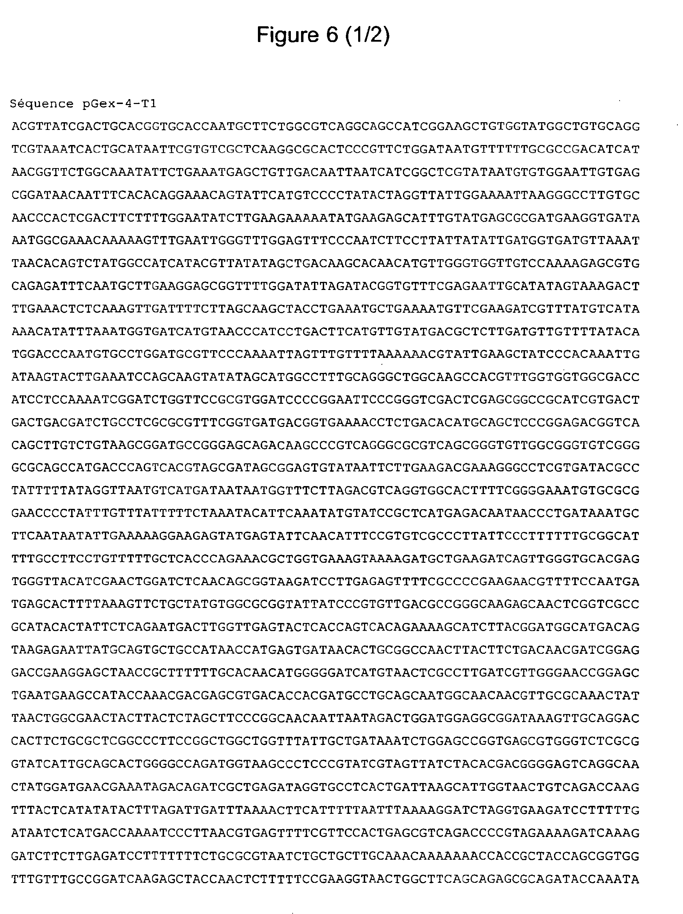

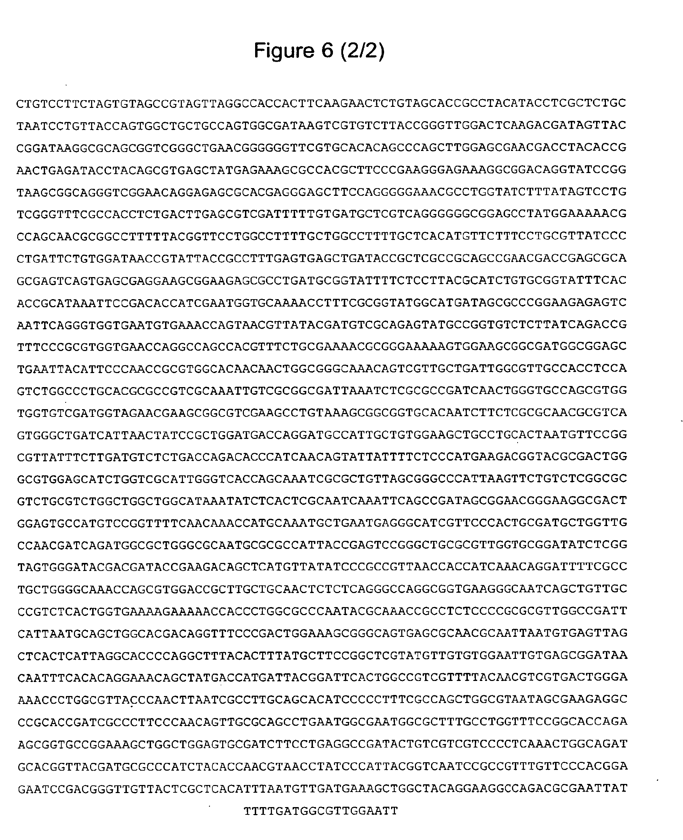

Figure 6 : séquence du plasmide de la série pGEX (SEQ ID NO 5).

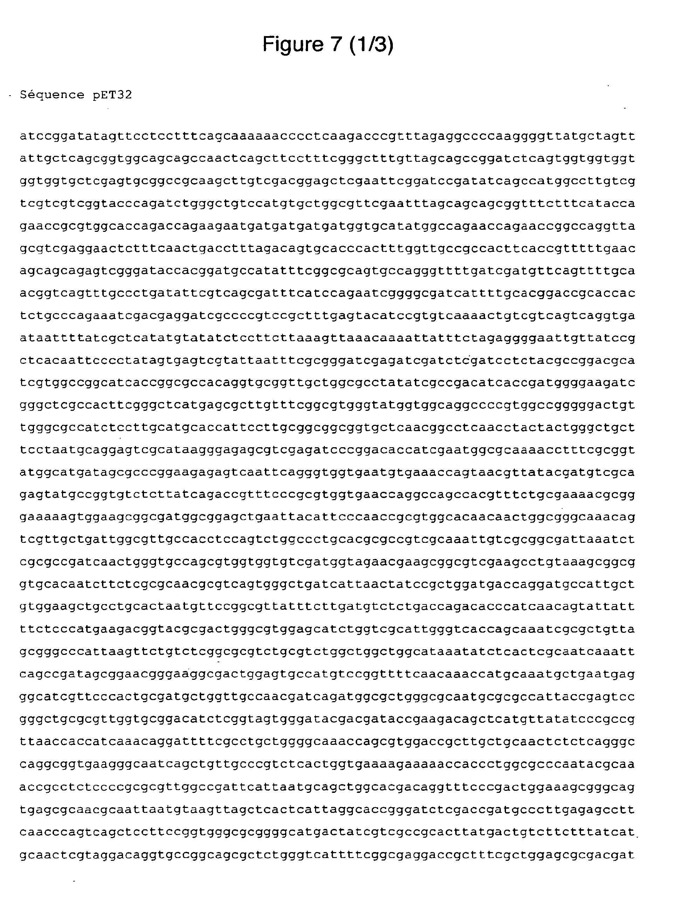

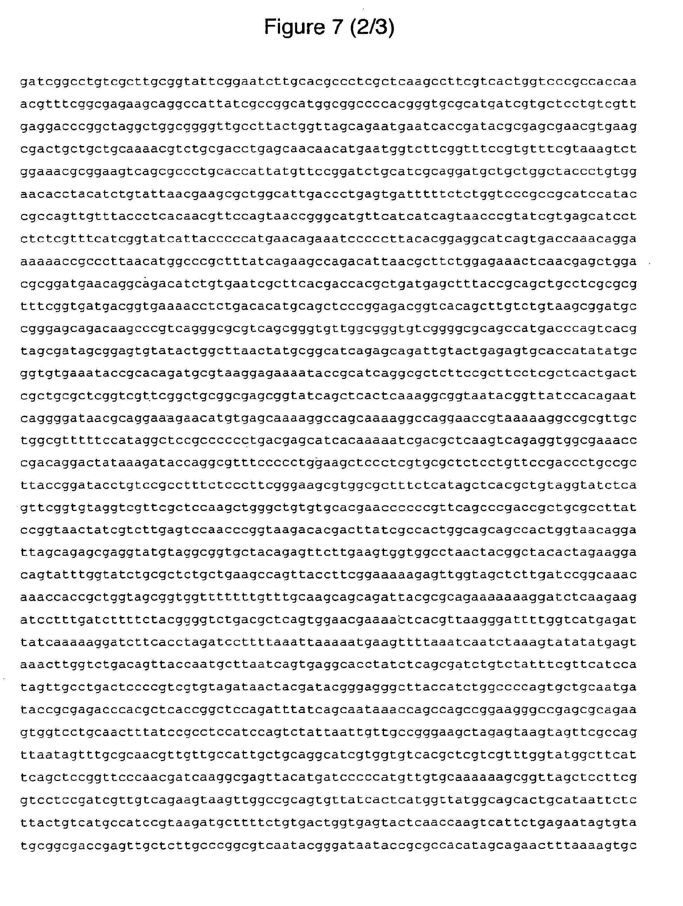

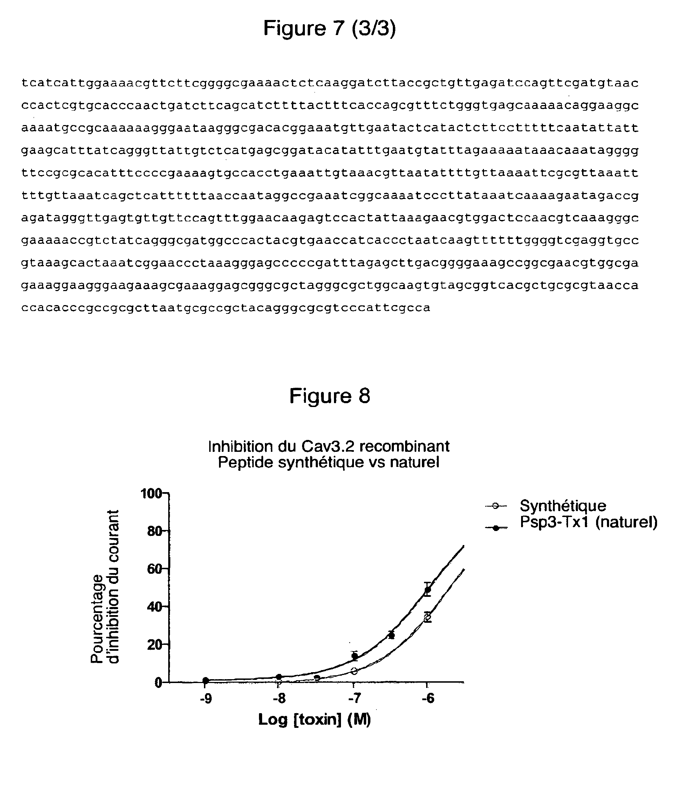

Figure 7 : séquence du plasmide de la série pET32 (SEQ ID NO 6).
Figure 8 est un diagramme représentant le pourcentage d'inhibition du courant généré par les canaux Caᵥ3.2 en fonction de le concentration de la toxine. L'ordonnée représente le pourcentage d'inhibition du courant généré par les canaux Caᵥ3.2 et l'abscisse le logarithme décimal de la concentration molaire de la toxine. La courbe avec les ronds pleins correspond à la courbe d'inhibition avec la toxine Psp3Tx1, la courbe avec les ronds creux correspond à la courbe d'inhibition avec la toxine synthétique.

### EXEMPLES

### Exemple 1 : caractérisation du peptide de l'invention

Le venin de PARAPHYSA SP3 a été dilué au 1000ième dans la solution extracellulaire utilisée pour l'analyse en patch clamp. Cette solution est de composition suivante (en mM): NaCl 135, TEACI 20, HEPES 10, CaCl₂ 2, MgCl₂ 1 (pH 7,3 avec TEAOH).

Des cellules HEK293 ont été transfectées avec un plasmide d'expression pcDNA3 contenant l'ADN complémentaire du canal calcique Cav3.2 humain et un gène rapporteur (CD8) à l'aide du réactif jetPEI™ (distribué par la société Q-biogen). Après 48 heures d'expression, les cellules transfectées ont été dispersées à l'aide de trypsine puis ensemencées à faible densité sur des boites de cultures pour l'analyse en patch clamp.

Les cellules positivement transfectées ont été repérées à l'aide de billes magnétiques recouvertes avec un anticorps anti-CD8 (Dynal) comme décrit précédemment dans Jimenez, C., Bourinet, E., Leuranguer, V., Richard, S., Snutch, T.P. and Nargeot, J. (2000) "Determinants of voltage-dependent inactivation affect Mibefradil block of calcium channels." Neuropharmacology, 39, 1-10 (**Réf 2**). Les enregistrements électrophysiologiques sont réalisés par la technique du « patch clamp » en configuration « whole cell » à l'aide d'un amplificateur Axopatch200B piloté par une interface Digidata et le logiciel pClamp9 (Molecular Devices). La solution d'enregistrement extracellulaire contenant 2mM calcium est décrite ci-dessus. Les enregistrements sont réalisés à l'aide de pipettes de borosilicate (Sutter Instruments) étirées pour avoir une résistance de 1,5 à 2,5mΩ. La solution intrapipette utilisée pour les enregistrements a la composition suivante : CsCl 130 mM, HEPES 10 mM, EGTA 10 mM, CaCl₂ 2 mM, MgCl₂ 1 mM, MgATP 4 mM, TrisGTP 0,3 mM (pH 7,3 avec NaOH). L'analyse des résultats est réalisée à l'aide des logiciels Clampfit (marque de commerce), Excel (marque de commerce) et GraphPad Prism (marque de commerce).

Les appareils utilisés dans cet exemple pour les enregistrements électrophysiologiques sont l'Amplificateur Axopatch200B (Molecular Device), Digidata 1300 (Molecular Device), le logiciel pClamp9 (Molecular Devices), l'amplificateur GeneClamp500 (Molecular Device), le microscope inversé Olympus IX70 (Olympus France), le micromanipulateur Sutter (Sutter Instruments). Les composés, réactifs, et cellules utilisés provenait pour les produits chimiques de la société Sigma-Aldrich France, les cellules utilisées étaient les cellules HEK293 (ATCC), les billes Dynal anti-CD8 de la société Invitrogen France, le Réactif jetPEI (marque de commerce) de la société Q-biogen France, le microorganisme Xenopus Laevis (CRBM - CNRS Montpellier France)

Pour visualiser les courants calciques de type T générés par les canaux Caᵥ3.2 recombinants exprimés dans les cellules transfectées, le protocole de stimulation avec suivant est utilisé : à partir d'un potentiel de repos de -100mV, une dépolarisation de 100 ms à un potentiel de -30 mV est appliquée toutes les 10 secondes. L'amplitude du courant généré par cette stimulation est mesurée en condition contrôle puis lors de l'application du venin de PARAPHYSA SP3. Pour ce faire, le venin de PARAPHYSA SP3 est aspiré dans une pipette identique à la pipette d'enregistrement. Cette pipette contenant le venin est connectée à un système de pression pneumatique permettant d'appliquer 1 à 2 microlitres de venin à proximité de la cellule enregistrée.

La caractérisation des fractions du venin de PARAPHYSA SP3 est effectuée dans un système d'expression différent. Le cDNA codant pour Cav3.2 humain est injecté dans des ovocytes de Xénope (Xenopus laevis) comme décrit dans Altier, C., Dubel, S.J., Barrère, C., Jarvis, S.E., Stotz, S.C., Spaetgens, R.L., Scott, J.D., Cornet, V., De Waard, M., Zamponi, G.W., Nargeot, J. and Bourinet, E. (2002) « Trafficking of L-type calcium channels mediated by the postsynaptic scaffolding protein AKAP79. » J. Biol. Chem., 277, 33598-33603 (**Réf 5**) et dans Dubel, S.J., Altier, C., Chaumont, S., Lory, P., Bourinet, E. and Nargeot, J. (2004) "Plasma Membrane Expression of T-type Calcium Channel {alpha}1 Subunits Is Modulated by High Voltage-activated Auxiliary Subunits." J Biol Chem, 279, 29263-29269 (**Réf 6**). Les courants sont enregistrés par la technique de voltage imposé par double microélectrode à l'aide d'un amplificateur GenClamp500 (marque de commerce) (Molecular Devices (marque enregistrée)) piloté par le logiciel pClamp9(marque de commerce). Les différentes fractions chromatographiques obtenues à partir du venin sont dissoutes dans la solution extracellulaire contenant (en mM): 5 BaOH₂, 25 TEAOH, 25 NaOH, 2 CsOH, 30 NMDG, 5 HEPES (pH à 7.3 avec de l'acide methanesulfonic). Les enregistrements sont réalisés dans une microcuve de 15µl. Les fractions (10µl) sont appliquées manuellement, directement sur l'ovocyte enregistré à l'aide d'une pipette.

Dés l'application de la toxine purifiée, le courant du canal Cav3.2 est fortement inhibé au bout de 30 secondes (Figure 2). Lors du lavage de l'effet de la toxine, la récupération de l'amplitude du courant de départ est obtenue après plusieurs minutes.

Par ailleurs, tel que montré sur la figure 4, l'application de l'extrait provoque un déplacement d'environ 20mV vers les potentiels positifs de la relation courant-potentiel. Ce déplacement est une caractéristique des toxines provoquant une inhibition du « senseur de voltage » des canaux ioniques. Cet effet classique des toxines d'araignée est décrit dans Winterfield JR, Swartz KJ. (2000) A hot spot for the interaction of gating modifier toxins with voltage-dependent ion channels. J Gen Physiol, 116(5):637-44.) (**Réf 10**). Il s'agit d'un blocage de la partie du canal qui détecte la variation de potentiel (le « voltage sensor »). La conséquence est que l'ouverture du canal est rendue plus difficile et nécessite une dépolarisation plus importante. Ainsi la relation courant potentiel est déplacée vers des potentiels plus positifs.

Il apparaît donc clairement que l'extrait comporte un antagoniste d'un canal de type T. Cette molécule est donc une nouvelle molécule permettant d'inhiber le signal douloureux.

### Exemple 2 : isolement et séquençage du peptide de l'invention

Les fractions de venin inhibant l'activité des canaux calciques tel que présenté dans l'exemple précédent ont été isolées et la recherche du composant responsable de cette inhibition a été effectuée par des étapes successives tests d'activité couplées avec les étapes de fractionnement

Le venin de PARAPHYSA SP. A été obtenu par stimulation électrique des chélicères de femelles adultes. Le venin a été collecté dans des microtubes, et lyophilisé

La préparation du venin pour les analyses d'activité et pour la séparation chromatographique a consisté en une dissolution du lyophilisat dans de l'eau ultra-pure à une dilution 10 fois de celle du volume de venin initial, une centrifugation (14 000 tours par minute, 20 min) et une filtration sur membrane de 0,45 µm (SJHVL04NS Millipore (marque de commerce)).

Purification du composant actif du venin par chromatographie : Dans une première étape, un aliquote de 100 µl de la dilution du venin (10 µl équivalents de venin brut) est fractionné par chromatographie liquide en phase inverse à haute performance (RP-HPLC) sur une colonne semi-préparative C8 (5C8MS, 10x250 mm, Waters). Le gradient utilisé est composé de eau + 0,1% acide trifluoroacétique (A) / acetonitrile + 0,1% acide trifluoroacétique (B). Le gradient est programmé comme suit: 0% B pendant 5 min, 0 à 60% B en 60 min, 60 à 90% B en 10 min, débit 2 mL/min. Les fractions sont collectées manuellement en sortie de colonne, en surveillant la variation de l'absorbance UV à 215 nm. Ces fractions sont ensuite lyophillisées puis leur activité est testée sur le canal calcium de type T comme décrit ci-dessus.

Une deuxième étape de purification est réalisée comme suit, sur les fractions de RP-HPLC actives : ces fractions sont analysées dans une deuxième étape de chromatographie d'échange de cations, sur une colonne Tosoh SP5PW (marque de commerce) (4,6 x 70 mm) (Tosoh), avec un gradient linéaire d'acétate d'ammonium dans l'eau (de 20 mM à 1 M en 50 min) avec un débit de 0.5 mL/min. Les fractions collectées sot lyophillisées puis leur activité est testée sur le canal calcium de type T comme décrit ci-dessus.

Après identification de la fraction active, la pureté et le poids moléculaire du peptide constituant la fraction active sont déterminés par spectrométrie de masse MALDI-TOF (Matrix-Assisted Laser Desorption/lonization Time-Of-Flight).

Les mesures de spectrométrie de masse sont réalisées par spectrométrie de masse MALDI-TOF sur un spectromètre de masse Voyager DE-Pro (marque de commerce) (Applied Biosystems (marque enregistrée)) en mode réflecteur. Les peptides sont mélangés avec la matrice acide α-cyano-4-hydroxycinnamique (α-CHCA, Sigma(marque enregistrée), 5 mg/mL dans 50:50:0,1 eau:acetonitrile:TFA) et analysés. Les spectres de masse sont calibrés avec des standards internes et analysés dans le logiciel Data Explorer (marque de commerce).

La séquence du peptide purifié est déterminée par séquençage automatique en utilisant un séquenceur en phase gazeuse (Applied Biosystems Procise) par dégradation d'Edman.

Avant analyse, les ponts disulfures du peptide purifié sont réduits par le DTT (Dithiothreitol 10 mM, 55°C, 45 min) et alkylés avec de l'IAA (acide lodoacétique, 50 mM, à 20°C, 30min) selon un protocole connu de l'homme du métier (voir John M. Walker. The Protein Protocols Handbook, Humana Press 1996 (ISBN 0-89603-339-2) (**Réf 14**). Le peptide réduit/alkylé est soumis à un dessalage par chromatographie RP-HPLC C18 sur une colonne Merck Chromolith Speed-Rod (marque de commerce) (0,46x50 mm). La séquence du peptide réduit/alkylé est ensuite déterminée par séquençage N-terminal automatique en phase gazeuse sur un séquenceur de peptides de modèle Applied Biosystems 477A (marque de commerce) (Applied Biosystems (marque enregistrée)).

La séquence peptidique obtenue est YCQKFLWTCDSERPCCEGLVCRLWCKIN (SEQ ID NO 1)

Cet exemple démontre donc clairement que la molécule antagoniste des canaux de type T est le peptide de séquence ID N°1.

Ce peptide représente donc une nouvelle molécule susceptible d'inhiber le signal douloureux.

### Exemple 3 : synthèse chimique du peptide de séquence SEQ ID NO 1.

La société Altergen (marque enregistrée) synthétise chimiquement le peptide de l'invention selon le protocole suivant : synthèse en phase solide selon la technique de Merifield, stratégie Fmoc.

L'activité inhibitrice du peptide identifié dans l'exemple 2 est testé in vitro sur différents canaux calciques de type T (résultats non fourni).

### Exemple 4 : synthèse chimique du peptide de séquence SEQ ID NO 1.

La société Genepep (marque enregistrée) a synthétise chimiquement le peptide de l'invention selon le protocole suivant : synthèse en phase solide selon technique de Merifield, stratégie Fmoc. (**Réf 4**)

La société Synprosis (marque enregistrée) a replié le peptide linéaire produit par la société Genepep selon le protocole suivant : incubation de 10µM du peptide linéaire Psp3-Tx1 dans un tampon contenant 1mM glutathion réduit (GSH), 1mM glutathion oxidé (GSSG), 100mM trishydroxyméthylaminométhane (TRIS) pH9, 25% glycérol. L'incubation a été réalisée pendant 24 heures sous agitation douce à température ambiante. La purification de la toxine Psp3-Tx1 synthétique a été réalisée en chromatographie HPLC en phase inverse sur une colonne RP-C18 Chromolith (marque enregistrée) en utilisant un gradient de 10 à 50% d'acétonitrile (ACN) dans une solution à 0,1% de trifluoroacetate (TFA).

La vérification de la masse du peptide synthétique replié a été réalisée par spectrométrie de masse donnant une valeur de 3398.0 daltons (Da) en accord avec la formation des 3 ponts disulfures entre les 6 cystéines.

### Exemple 5: caractérisation du peptide synthétique de l'invention produit dans l'exemple 4.

L'activité inhibitrice du peptide synthétique de l'exemple 4 a été testée in vitro sur le canal Caᵥ3.2. Une comparaison de l'inhibions avec le peptide naturel (Psp3-Tx1) à été réalisée dans les même conditions. Le peptide synthétique et le peptide naturel (toxine naturelle) ont été dilués à une concentration stock de 10⁻³ molaire dans de l'eau distillée. A partir de cette concentration stock, les peptides ont été dilués dans une solution correspondant à la solution extracellulaire utilisée pour l'analyse en patch clamp. Cette solution est de composition suivante (en mM): NaCl 135, TEACI 20, HEPES 10, CaCl₂ 2, MgCl₂ 1 (pH 7,3 avec TEAOH). Les concentrations testées ont été les suivantes : pour le peptide synthétique : 10⁻⁸, 10⁻⁷, et 10⁻⁶ molaire; pour le peptide naturel : 10⁻⁹, 10⁻⁸, 3.10⁻⁸, 10⁻⁷, 3.10⁻⁷, et 10⁻⁶ molaire.

Des cellules HEK293 (ATCC) exprimant de manière stable la séquence d'ADN complémentaire du canal calcique Cav3.2 humain ont été utilisées. A chaque passage de la lignée cellulaire, des cellules ont été ensemencées à faible densité sur des boites de cultures pour l'analyse en « patch clamp » (électrophysiologie moléculaire).

Les cellules positivement transfectées ont été repérées à l'aide de billes magnétiques recouvertes avec un anticorps anti-CD8 (Dynal) comme décrit précédemment dans Jimenez, C., Bourinet, E., Leuranguer, V., Richard, S., Snutch, T.P. and Nargeot, J. (2000) "Determinants of voltage-dependent inactivation affect Mibefradil block of calcium channels." Neuropharmacology, 39, 1-10 (**Réf 2**).

Les enregistrements électrophysiologiques sont réalisés par la technique du « patch clamp » en configuration « whole cell » à l'aide d'un amplificateur Axopatch200B piloté par une interface Digidata et le logiciel pClamp10 (Molecular Devices). La solution d'enregistrement extracellulaire contenant 2mM calcium est décrite ci-dessus.

Les enregistrements sont réalisés à l'aide de pipettes de borosilicate (Sutter Instruments) étirées pour avoir une résistance de 1,5 à 2,5mΩ.

La solution intrapipette utilisée pour les enregistrements est de composition suivante : CsCl 130 mM, HEPES 10 mM, EGTA 10 mM, CaCl₂ 2 mM, MgCl₂ 1 mM, MgATP 4 mM, TrisGTP 0,3 mM (pH 7,3 avec NaOH). L'analyse des résultats est réalisée à l'aide des logiciels Clampfit (marque de commerce), Excel (marque de commerce) et GraphPad Prism (marque de commerce).

Les appareils utilisés dans cet exemple pour les enregistrements électrophysiologiques sont l'Amplificateur Axopatch200B (Molecular Device), Digidata 1440 (Molecular Device), le logiciel pClamp10 (Molecular Devices), l'amplificateur GeneClamp500 (Molecular Device), le microscope inversé Olympus IX70 (Olympus France), le micromanipulateur Sutter (Sutter Instruments). Les composés, réactifs, et cellules utilisés provenaient pour les produits chimiques de la société Sigma-Aldrich France, les cellules utilisées étaient les cellules HEK293 (ATCC), les billes Dynal anti-CD8 de la société Invitrogen France, le Réactif jetPEI (marque de commerce) de la société Q-biogen France.

Pour visualiser les courants calciques de type T générés par les canaux Caᵥ3.2 recombinants exprimés dans les cellules transfectées, le protocole de stimulation avec suivant est utilisé : à partir d'un potentiel de repos de -100mV, une dépolarisation de 100 ms à un potentiel de -30 mV est appliquée toutes les 10 secondes.

L'amplitude du courant généré par cette stimulation est mesurée en condition contrôle puis lors de l'application de concentration croissantes du peptide synthétique de l'exemple 4, ou de concentrations croissantes de la toxine native Psp3-Tx1. Pour ce faire, les solutions contenant les différentes concentrations du peptide synthétique ou de la toxine Psp3-Tx1 sont réparties dans des tubes de perfusion connectés à un collecteur permettant d'appliquer ces différentes solutions à proximité de la cellule enregistrée.

L'activité inhibitrice de chaque concentration du peptide synthétique ou de la toxine native est ainsi appréhendée en mesurant l'amplitude du courant CaV3.2 de la cellule enregistrée au plateau de l'effet.

Les effets inhibiteurs du peptide synthétique et du peptide naturel ont été présentés sous forme de pourcentage de bloc à chaque concentration pour construire des courbes « effet dose » présentés dans la figure 8. Ces courbes « effet dose » ont été analysées avec le logiciel GrapPad Prism (marque du commerce) avec la fonction d'analyse sigmoïde « log(antagonist) vs. response - variable slope » permettant de calculer la dose inhibant 50% du courant (EC50) ainsi que la pente de la courbe correspondant ou nombre de Hill.

La figure 8 représente les courbes dose-réponses pour le peptide naturel (Psp3Tx1) (courbe avec des ronds noirs pleins) et le peptide synthétique (courbe avec les ronds noirs creux).

Ainsi dans la figure 8, l'EC50 pour la toxine naturelle est de 1.1µM avec un nombre de Hill de 0.87 (nombre de cellules analysées est de 6), et l'EC50 pour le peptide synthétique est de 2.01 µM avec un nombre de Hill de 0.89 (nombre de cellules analysées égale à 20).

Les résultats montrent donc que le peptide synthétique et le peptide natif inhibent les courants du canal Caᵥ3.2. Les résultats sont décrits avec un coefficient de Hill égale à environ 1, impliquant une interaction 1 :1 entre le canal et les peptides.

Cet exemple démontre donc clairement que le peptide de séquence ID NO 1, extrait du venin d'araignée comme présenté dans l'exemple 1 et/ou synthétisé chimiquement, inhibe les courants du canal Caᵥ3.2

### Exemple 6 : effet analgésique du peptide de l'invention.

Des souris sont utilisées afin d'observer l'effet analgésique du peptide de l'invention vis-à-vis de douleurs induites par des stimuli thermiques.

Les souris sont des souris C57BL/6J sauvages et des souris C57BL/6J CaV3.2-/-. Le nombre total de souris est de 10 sauvages et 10 CaV3.2-/-.

Deux groupes de souris (C57BL/6J) sont utilisés, un groupe dans lequel on a injecté par voie intrathécale une solution physiologique comprenant le peptide de l'invention à une concentration de 10 et 100 nM et un deuxième groupe (groupe témoin) ne recevant que l'injection intrathécale de la solution physiologique seule. Les souris sont ensuite soumises au test de l'immersion de la queue dans un bain marie à 46°C. Cette expérience a pour but de déterminer le temps au bout duquel les souris réagissent au chaud douloureux montrant ainsi le seuil d'apparition du signal douloureux lié à la perception de la chaleur. La même expérience est réalisée en parallèle sur deux autres groupes de souris C57BL/6J dépourvues du gène codant pour le canal calcique de type-T codé par la sous unité CaV3.2. Cette expérience a pour but de montrer la sélectivité du peptide de l'invention.

Les résultats montrent une différence significative du temps de réaction à la chaleur entre le groupe de souris dans lequel le peptide a été injecté et le groupe de souris témoin. Le temps de réaction à la chaleur étant significativement plus long chez les souris qui reçoivent le peptide de séquence SEQ ID NO 1.

Des souris sont utilisées afin d'observer l'effet analgésique du peptide de l'invention vis à vis de stimuli mécaniques.

Les souris sont des souris C57BL/6J sauvages et des souris C57BL/6J CaV3.2-/-. Le nombre total de souris est de 10 sauvages et 10 CaV3.2-/-.

Deux groupes de souris (C57BL/6J) sont utilisés, un groupe dans lequel on a injecté par voie intrathécal une solution physiologique comprenant le peptide de l'invention à une concentration de 10 et 100 nM et un deuxième groupe (groupe témoin) ne recevant que l'injection intrathécal de la solution physiologique seule. Les souris sont ensuite soumises à l'expérience de stimulation mécanique par les soies de Von Frey. Cette expérience a pour but de déterminer le seuil de réaction à un stimulus mécanique d'intensité croissante appliqué sur la voûte plantaire. Le seuil de réaction des animaux est corrélé à l'apparition du signal douloureux lié à la perception de la pression mécanique. La même expérience est réalisée en parallèle sur deux autres groupes de souris C57BL/6J dépourvues du gène codant pour le canal calcique de type-T codé par la sous unité CaV3.2. Cette expérience a pour but de montrer la sélectivité du peptide de l'invention.

Les résultats montrent une différence significative de seuil de perception à la pression mécanique entre le groupe de souris dans lequel le peptide a été injecté et le groupe de souris témoin. Le seuil de pression étant significativement plus élevé chez les souris qui reçoivent le peptide de séquence SEQ ID NO 1.

Le peptide de l'invention a donc un effet analgésique attendu vis-à-vis de stimuli douloureux thermiques et mécaniques.

Cet exemple démontre donc que le peptide de séquence SEQ ID NO 1, identifié dans l'exemple 2, est une nouvelle molécule pour le traitement de la douleur.

### Exemple 7: comparaison de l'effet analgésique du peptide de l'invention avec celui d'un analgésique de référence.

Les souris et le protocole expérimental utilisé dans le présent exemple sont identiques à ceux décrits dans l'exemple 4.

Deux groupes de souris supplémentaires sont testés :
- un groupe dans lequel a été injecté par voie intrathécale une dose de morphine (1µg/kg), et
- un groupe dans lequel a été le peptide de séquence SEQ ID NO 1 (10nM).

### Exemple 8 : effet antihypertenseur du peptide de l'invention.

Les souris utilisées dans le présent exemple sont identiques à ceux décrits dans l'exemple 4.

Des souris sont utilisées afin d'observer l'effet antihypertenseur du peptide de l'invention. Deux groupes de souris (C57BL/6J) sont utilisés, un groupe dans lequel on injecte par voie intraveineuse une solution physiologique comprenant le peptide de l'invention à une concentration de 10 et 100 nM et un deuxième groupe (groupe témoin) ne recevant que l'injection intraveineuse de la solution physiologique seule. La mesure de la pression artérielle est réalisée par le principe de la méthode du manchon caudal (Plehm, R., Barbosa, M.E. and Bader, M. (2006) Animal models for hypertension/blood pressure recording. Methods Mol Med, 129, (**Réf 15**)).

### Exemple 9 : effet antiprolifératif du peptide de l'invention.

Des cellules cancéreuses de prostate LNCAP sont utilisées afin d'observer l'effet antiprolifératif du peptide de l'invention.
Le milieu de culture utilisé est le milieur RPMI 1640 (BioWhittaker SA, Verviers, Belgium), supplémenté de 5 mM L-glutamine (Sigma-Aldrich, L'Isle d'Abeau, France) et de 10% FBS (Applera France SA, Courtaboeuf, France) comme indiqué dans la le document Gackière F, Bidaux G, Delcourt P, Van Coppenolle F, Katsogiannou M, Dewailly E, Bavencoffe A, Van Chuoï-Mariot MT, Mauroy B, Prevarskaya N, Mariot P. (2008) CaV3.2 T-type calcium channels are involved in calcium-dependent secretion of neuroendocrine prostate cancer cells. J Biol Chem, 283(15):10162-73. (**Réf 16**)

Deux conditions de cellules sont utilisées :
- une condition dans laquelle les cellules sont cultivées dans différents essais en présence du peptide de l'invention à des concentrations de 10, 30, 100, 300, 1000 nM, et
- une deuxième condition (témoin) sans peptide.

La mesure de la prolifération cellulaire est réalisée par la méthode CellTrace (marque de commerce) CFSE (Invitrogen (marque de enregistrée)) basée sur l'incorporation du traceur fluorescent CFSE par les cellules cultivées et d'une mesure par cytomètre de flux des cellules marquées

### Listes des références

**Ref 1** Bourinet, E., et al., Silencing of the CaV3.2 T-type calcium channel gene in sensory neurons demonstrates its major role in nociception. Embo J, 2005. 24(2): p. 315-24.
**Ref 2** Jimenez, C., Bourinet, E., Leuranguer, V., Richard, S., Snutch, T.P. and Nargeot, J. (2000) Determinants of voltage-dependent inactivation affect Mibefradil block of calcium channels. Neuropharmacology, 39, 1-10.
**Ref 3** Sambrook, Fritsch and Maniatis, Molecular cloning, A laboratory manual, second edition, Cold spring Harbor Laboratory Press, 1989
**Ref 4** Fmoc solid Phase peptide synthesis, a practical approach », publié par W.C. Chan et P. D.White, Oxford university press, 2000
**Ref 5** Altier, C., Dubel, S.J., Barrère, C., Jarvis, S.E., Stotz, S.C., Spaetgens, R.L., Scott, J.D., Cornet, V., De Waard, M., Zamponi, G.W., Nargeot, J. and Bourinet, E. (2002) Trafficking of L-type calcium channels mediated by the postsynaptic scaffolding protein AKAP79. J. Bio/. Chem., 277, 33598-33603.
**Ref 6** Dubel, S.J., Altier, C., Chaumont, S., Lory, P., Bourinet, E. and Nargeot, J. (2004) Plasma Membrane Expression of T-type Calcium Channel {alpha}1 Subunits Is Modulated by High Voltage-activated Auxiliary Subunits. J Biol Chem, 279, 29263-29269.
**Ref 7** Pharmacie galénique de A. LEHIR (Ed. Masson, 1992 (6 eme édition)
**Ref 8** Taylor, J.T., Zeng, X.B., Pottle, J.E., Lee, K., Wang, A.R., Yi, S.G., Scruggs, J.A., Sikka, S.S. and Li, M. (2008) Calcium signaling and T-type calcium channels in cancer cell cycling. World J Gastroenterol, 14, 4984-4991.
**Ref 9** Chen, C.C., Lamping, K.G., Nuno, D.W., Barresi, R., Prouty, S.J., Lavoie, J.L., Cribbs, L.L., England, S.K., Sigmund, C.D., Weiss, R.M., Williamson, R.A., Hill, J.A. and Campbell, K.P. (2003) Abnormal coronary function in mice deficient in alpha1H T-type Ca2+ channels. Science, 302, 1416-1418.
**Ref 10** Winterfield JR, Swartz KJ. (2000) A hot spot for the interaction of gating modifier toxins with voltage-dependent ion channels. J Gen Physiol, 116(5):637-44.)
**Ref 11** Panner A, Cribbs LL, Zainelli GM, Origitano TC, Singh S, Wurster RD. (2005) Variation of T-type calcium channel protein expression affects cell division of cultured tumor cells. Cell Calcium, 37(2):105-19
**Ref 12** Lu F, Chen H, Zhou C, Liu S, Guo M, Chen P, Zhuang H, Xie D, Wu S. (2008) T-type Ca2+ channel expression in human esophageal carcinomas: a functional role in proliferation Cell Calcium. 43(1):49-58
**Ref 13** Taylor JT, Huang L, Pottle JE, Liu K, Yang Y, Zeng X, Keyser BM, Agrawal KC, Hansen JB, Li M (2008) Selective blockade of T-type Ca2+ channels suppresses human breast cancer cell proliferation.. Cancer Lett, 18;267(1):116-24
**Ref 14** John M. Walker. The Protein Protocols Handbook, Humana Press 1996 (ISBN 0-89603-339-2)
**Ref 15** Plehm, R., Barbosa, M.E. and Bader, M. (2006) Animal models for hypertension/blood pressure recording. Methods Mol Med, 129
**Ref 16** Gackière F, Bidaux G, Delcourt P, Van Coppenolle F, Katsogiannou M, Dewailly E, Bavencoffe A, Van Chuoï-Mariot MT, Mauroy B, Prevarskaya N, Mariot P. (2008) CaV3.2 T-type calcium channels are involved in calcium-dependent secretion of neuroendocrine prostate cancer cells. J Biol Chem, 283 (15):10162-73.
**Ref 17** Han KK, Martinage A (1992) Post-translational chemical modification(s) of proteins. Int J Biochem. 24(1):19-28. ; Walsh G, Jefferis R (2006) Post-translational modifications in the context of therapeutic proteins Nature Biotechnology - 24, 1241 - 1252
**Ref 18** Escoubas P. (2006) Molecular diversification in spider venoms: a web of combinatorial peptide libraries. Mol Divers. 10(4):545-54).

## Revendications

1. Peptide de séquence en acides aminés
YCQKFLWTCDSERPCCEGLVCRLWCKIN (SEQ ID NO :1)

2. Acides nucléiques codant pour le peptide défini dans la revendication 1.

3. Acide nucléique selon la revendication 2, dans lequel la séquence de l'acide nucléique est dans lesquelles A est adénosine, C est cytidine, G est guanosine T est thymidine et H est A ou C ou T, M est A ou C, N est A ou C ou G ou T, R est A ou G, S est C ou G, W est A ou T et Y est C ou T

4. Système d'expression comprenant un plasmide et/ou un vecteur d'expression comprenant un acide nucléique selon la revendication 2.

5. Peptide antagoniste d'un canal calcique de type T de séquence en acide aminés :
YCQKFLWTCDSERPCCEGLVCRLWCKIN (SEQ ID NO :1).

6. Peptide antagoniste d'un canal calcique de type T selon la revendication 5, ledit canal est un canal choisi dans le groupe comprenant le canal Cav3.2 et le canal Cav3.1.

7. Peptide selon la revendication 1 pour son utilisation comme médicament.

8. Peptide selon la revendication 1 pour son utilisation dans le traitement de la douleur.

9. Peptide selon la revendication 1 pour son utilisation comme médicament analgésique.

10. Procédé de synthèse du peptide selon la revendication 1, **caractérisé en ce qu'**il comprend une étape de synthèse chimique sur support solide ou une étape de synthèse par recombinaison génétique.

## Patentansprüche

1. Peptid mit der Aminosäuresequenz
YCQKFLWTCDSERPCCEGLVCRLWCKIN (SEQ ID NO:1)

2. Nukleinsäuren, die für das in Anspruch 1 definierte Peptid kodieren.

3. Nukleinsäure nach Anspruch 2, in der die Nukleinsäuresequenz folgende ist worin A für Adenosin, C für Cytidin, G für Guanosin, T für Thymidin steht und H A oder C oder T ist, M A oder C ist, N A oder C oder G oder T ist, R A oder G ist, S C oder G ist, W A oder T ist und Y C oder T ist.

4. Expressionssystem, umfassend ein Plasmid und/oder einen Expressionsvektor, umfassend eine Nukleinsäure nach Anspruch 2.

5. Peptid als Antagonist eines Kalziumkanals vom T-Typ der Aminosäuresequenz :
YCQKFLWTCDSERPCCEGLVCRLWCKIN (SEQ ID NO :1).

6. Peptid als Antagonist eines Kalziumkanals vom T-Typ nach Anspruch 5, wobei der Kanal ein Kanal ist ausgewählt aus der Gruppe umfassend den Cav3.2-Kanal und Cav3.1-Kanal.

7. Peptid nach Anspruch 1 zu seiner Verwendung als Medikament.

8. Peptid nach Anspruch 1 zu seiner Verwendung in der Behandlung von Schmerzen.

9. Peptid nach Anspruch 1 zu seiner Verwendung als Schmerzmittel.

10. Verfahren zur Synthese des Peptids nach Anspruch 1, **dadurch gekennzeichnet, dass** es einen Schritt der chemischen Synthese auf einem festen Träger oder einen Syntheseschritt durch genetische Rekombination umfasst.

## Claims

1. A peptide of amino acid sequence
YCQKFLWTCDSERPCCEGLVCRLWCKIN (SEQ ID NO 1).

2. A nucleic acid coding for the peptide according to claim 1.

3. The nucleic acid according to claim 2, wherein the nucleic acid sequence is wherein A is adenosine, C is cytidine, G is guanosine T is thymidine and H is A or C or T, M is A or C, N is A or C or G or T, R is A or G, S is C or G, W is A or T and Y is C or T.

4. An expression system comprising a plasmid and/or an expression vector comprising a nucleic acid according to claim 2.

5. Peptide antagonist of a T-type calcium channel of amino acid sequence:
YCQKFLWTCDSERPCCEGLVCRLWCKIN (SEQ ID NO 1).

6. Peptide antagonist of a T-type calcium channel according to claim 5, said channel is a channel selected from the group comprising the Cav3.2 channel and the Cav3.1 channel.

7. Peptide according to claim 1 for its use as a medicament.

8. Peptide according to claim 1 for its use in the treatment of pain.

9. Peptide according to claim 1 for its use as analgesic medicament.

10. A method of synthesis of the peptide according to claim 1, **characterized in that** it comprises a step of chemical synthesis on a solid substrate or a step of synthesis by genetic recombination.
